(19) Europäisches Patentamt
European Patent Office
Office européen des brevets

(11) **EP 1 875 380 B1**

(12) **EUROPÄISCHE PATENTSCHRIFT**

(45) Veröffentlichungstag und Bekanntmachung des Hinweises auf die Patenterteilung:
**28.06.2017 Patentblatt 2017/26**

(21) Anmeldenummer: **06722696.9**

(22) Anmeldetag: **28.03.2006**

(51) Int Cl.:
*A61B 5/00* (2006.01)          *G01F 1/708* (2006.01)
*G01S 11/14* (2006.01)         *A61B 5/087* (2006.01)
*G01F 1/66* (2006.01)

(86) Internationale Anmeldenummer:
**PCT/DE2006/000544**

(87) Internationale Veröffentlichungsnummer:
**WO 2006/105761 (12.10.2006 Gazette 2006/41)**

(54) **VERFAHREN ZUR BESTIMMUNG DER ZEITLICHEN LAGE EINES WELLENPAKETS SOWIE FLUSSMESSGERÄT**

METHOD FOR DETERMINING THE TEMPORAL POSITION OF A WAVE PACKET AND FLOW MEASURING DEVICE

PROCÉDÉ POUR DÉTERMINER LA POSITION TEMPORELLE D'UN PAQUET D'ONDES ET APPAREIL DE MESURE DE FLUX

(84) Benannte Vertragsstaaten:
**CH DE FR GB LI**

(30) Priorität: **04.04.2005 DE 102005015456**

(43) Veröffentlichungstag der Anmeldung:
**09.01.2008 Patentblatt 2008/02**

(73) Patentinhaber: **CareFusion Germany 234 GmbH**
**97204 Höchberg (DE)**

(72) Erfinder:
• **GLASER, Eckard**
**97218 Gerbrunn (DE)**
• **SCHEDER, Daniel**
**97082 Würzburg (DE)**

(74) Vertreter: **Hellmich, Wolfgang**
**European Patent and Trademark Attorney**
**Lortzingstrasse 9 / 2. Stock**
**81241 München (DE)**

(56) Entgegenhaltungen:
EP-A- 0 797 105       GB-A- 2 275 108
US-A- 5 419 326       US-A1- 2002 143 479
US-B1- 6 279 379      US-B1- 6 305 233

**EP 1 875 380 B1**

**Beschreibung**

Die Erfindung bezieht sich auf die Signalaufbereitung, um einen Laufzeitunterschied exakt zu messen. Die Erfindung bezieht sich ferner auf Flussmessgeräte, insbesondere Flussmessgeräte zum Erfassen des Luftflusses beim Ein- und Ausatmen, die den Mitnahmeeffekt von Ultraschall ausnutzen. Solche Flussmessgeräte werden auch als Spirometer bezeichnet. Ein Verfahren zur Bestimmung der zeitlichen Lage eines Wellenpakets in einem Flussmessgerät, insbesondere mit Spirometern gemäß dem Oberbegriff des Anspruchs 1 sowie ein Flussmessgerät gemäß dem Oberbegriff des Anspruchs 8 ist beispielsweise aus der GB 2 275 108 A bekannt.

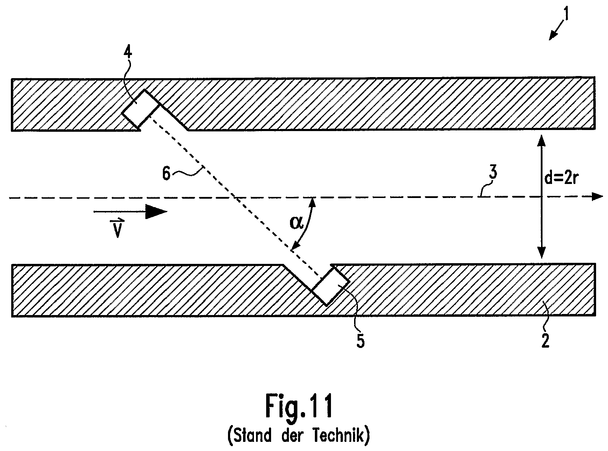

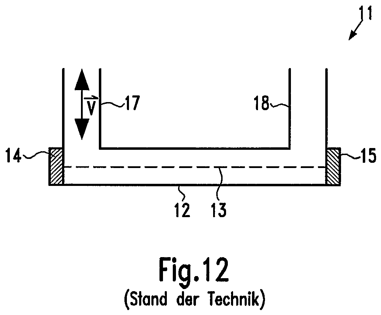

Es sind eine Reihe von Geräten bekannt, die den Mitnahmeeffekt von Schallwellen in strömenden Medien, insbesondere Luft ausnutzen. Hierbei gibt es im wesentlichen zwei Bauformen, die in den Figuren 11 und 12 dargestellt sind.

Das in Figur 11 dargestellte Flussmessgerät 1 umfasst ein Rohr 2 sowie einen ersten Ultraschallwandler 4 und einen zweiten Ultraschallwandler 5. Das Rohr hat einen Durchmesser d und eine Achse 3. Die beiden Ultraschallwandler 4 und 5 sind auf einer zweiten Achse 6 angeordnet, sodass jeder der beiden Wandler den vom jeweils anderen Wandler erzeugten Ultraschall auffängt. Die zweite Achse 6 schließt mit der Achse 3 des Rohres einen Winkel $\alpha$ ein. Bei ruhendem Medium im Rohr 2 gilt für die Schalllaufzeiten $t_{45}$ und $t_{54}$ von Ultraschallwandler 4 zu Ultraschallwandler 5 bzw. von Ultraschallwandler 5 zu Ultraschallwandler 4

$$t_{45} = t_{54} = \frac{L}{c} \tag{1}$$

wobei L der Abstand der beiden Ultraschallwandler und c die Schallgeschwindigkeit im ruhenden Medium bezeichnen.

Durch eine Strömung im Rohr 2 nach rechts verkürzt sich die Schalllaufzeit $t_{45}$ von Ultraschallwandler 4 zu Ultraschallwandler 5. Gleichzeitig verlängert sich die Schalllaufzeit $t_{54}$ von Ultraschallwandler 5 zu Ultraschallwandler 4. Es gilt für ein rechteckförmiges Strömungsprofil:

$$t_{45} = \frac{L}{c + v \cdot \cos(\alpha)} \tag{2}$$

$$t_{54} = \frac{L}{c - v \cdot \cos(\alpha)} \tag{3}$$

Hierbei bezeichnet v die Geschwindigkeit des strömenden Mediums nach rechts. Durch Einführung des Laufzeitunterschieds $\Delta t$ und der mittleren Laufzeit $t_0$

$$\Delta t \equiv t_{54} - t_{45} \tag{4}$$

$$t_0 \equiv \frac{t_{54} + t_{45}}{2} \tag{5}$$

erhält man für die Strömungsgeschwindigkeit v:

$$v = \frac{c}{2\cos(\alpha)} \frac{\Delta t}{t_0} \tag{6}$$

Aus (2) und (3) kann die Schallgeschwindigkeit im ruhenden Medium berechnet werden:

$$c = \frac{L}{t_0 - \frac{1}{4}\frac{\Delta t^2}{t_0}} \tag{7}$$

Durch Einsetzen von Formel (7) in (6) erhält man:

$$\dot{V} = C_1 \frac{L}{2\cos(\alpha)} \frac{\Delta t}{t_0{}^2 - \frac{1}{4}\Delta t^2} \tag{8}$$

Hierbei ist die Konstante $C_1$ der Umrechnungsfaktor zwischen Strömungsgeschwindigkeit v und Volumenstrom V. In die Konstante $C_1$ geht im wesentlichen die Querschnittfläche von Rohr 2 ein, wobei r den Radius von Rohr 2 bezeichnet.

$$C_1 = C_2 \pi r^2 \tag{9}$$

Die Konstante $C_2$ ist für ein rechteckförmiges Strömungsprofil 1. In realiter bildet sich im Rohr ein Geschwindigkeitsprofil aus, sodass die Geschwindigkeit des strömenden Mediums von der Position im Rohr, vom Volumenfluss und der Viskosität des strömenden Mediums abhängt. Für typische Volumenflüsse, das Medium Luft und Rohrdurchmesser von zwei bis drei Zentimetern bildet sich an der Rohrwand ein laminarer oder turbulenter Strömungsbereich und in Rohrmitte ein turbulenter Strömungsbereich aus. Je größer der Volumenfluss, desto kleiner der laminare Bereich und desto größer der turbulente Bereich. Dieses Strömungsprofil ist für Nichtlinearitäten in der Sensorkennlinie verantwortlich.

Aus den Laufzeiten $t_{45}$ und $t_{54}$ oder dem Laufzeitunterschied $\Delta t$ und der mittleren Laufzeit $t_0$ kann der Fluss im Rohr berechnet werden. Dieses Messprinzip ist unter anderem in EP 0 051 293 A1, EP 0 243 515 A1, EP 0 597 060 B1, CH 669 463 A5, WO 00/26618 A1 und EP 0 713 080 A1 beschrieben. Je kleiner der Winkel $\alpha$, desto größer ist der Laufzeitunterschied bei gleichem Fluss.

Das in Figur 12 dargestellte bekannte Flussmessgerät 11 umfasst ein U-förmiges Rohr, das aus einem waagrechten Schenkel 12 und zwei senkrechten Schenkeln 17 und 18 besteht. Darüber hinaus umfasst das Flussmessgerät 11 Ultraschallwandler 14 und 15, die auf einer Achse 13 angeordnet sind. Die Achse 13 ist gleichzeitig Achse von waagrechtem Schenkel 12. Vorteilhaft an diesem Aufbau ist, dass die Flussrichtung parallel zur Achse der beiden Ultraschallwandler verläuft und somit der Winkel $\alpha$ 0° und damit $\cos(\alpha)=1$ wird. Nachteilig ist der komplizierte Aufbau sowie Wirbel, die in den Übergangsbereich inzwischen den senkrechten und dem waagrechten Schenkel entstehen. Eine solche Bauformen ist beispielsweise in WO 90/05283 A1 oder EP 1 279 368 A2 beschrieben.

Unabhängig von der Bauformen ist bekannt (z. B. WO 90/05283, EP 1 279 368 A2), die Ultraschallwandler mit akustischen Impedanzwandlern zu versehen, um die Schallübertragung zwischen dem strömenden Medium und dem Ultraschallwandler und umgekehrt zu verbessern.

Der Zustand und die Zusammensetzung des zu messenden Gases ändert sich während der Atmung. Die Umgebungsbedingungen werden als Ambient Temperature Pressure (ATP) bezeichnet, die in Body Temperature Pressure Saturated (BTPS) umgerechnet werden können. Ausgeatmetes Gas hat eine Temperatur von etwa 34°C bis 37°C und ist vollständig mit Wasserdampf gesättigt, entspricht also BTPS.

Darüber hinaus ist die Rundsing-Technik (Englisch: sing-around technique) beispielsweise aus der EP 0 713 080 A1 bekannt. Bei der Rundsing-Technik werden wiederholt Schallimpulse von einem Ultraschallwandler zum anderen Ultraschallwandler gesendet. Sobald ein Impuls empfangen wird, wird der nächste Impuls gesendet. Nach 1 bis 200 Impulsen wird die Richtung umgekehrt. Bei diesem Verfahren wird die Zeitauflösung der Schalllaufzeit auf Kosten der Messrate erhöht. Dies ist insbesondere bei Medien mit hoher Schallgeschwindigkeit, insbesondere Flüssigkeiten, oder falls die Laufzeit aus technischen Gründen nur mit starkem Rauschen behaftet bestimmt werden kann, von Bedeutung.

Zur Analyse insbesondere von sich zeitlich verändernden Funktionen oder Signalen sind beispielsweise die Korrelationsanalyse, die Fourieranalyse oder -transformation, die Kurzzeitfouriertransformation sowie die Wavelettransformation bekannt.

Der Korrelationskoeffizient $\varphi$ zweier Funktionen f(t) und g(t), die von der Zeit t abhängen und im Intervall von $T_1$ bis $T_2$ definiert sind, ist definiert als:

$$\varphi = \frac{\int\limits_{T_1}^{T_2} f(t)g(t)dt}{\sqrt{\int\limits_{T_1}^{T_2} f^2(t)dt \int\limits_{T_1}^{T_2} g^2(t)dt}} \tag{10}$$

Der Korrelationskoeffizient kann als Maß für die Ähnlichkeit zweier Funktionen interpretiert werden.

Ferner ist die Kreuzkorrelationsfunktion $\Phi$ (KKF) definiert als zeitlicher Mittelwert des inneren Produkts der beiden um die Zeit $\tau$ gegeneinander verschobenen Funktionen f(t) und g(t):

$$\Phi(\tau) = \frac{1}{T_2 - T_1} \int\limits_{T_1}^{T_2} f(t)g(t+\tau)dt \tag{11}$$

Periodische Funktionen mit der Periodendauer ($T_2$-$T_1$) können durch Fourier-Transformation vollständig als gewichtete Summe von Sinus- und Kosinusfunktionen dargestellt werden. Die Fourierkoeffizienten können als Korrelationsfaktor einer periodischen Funktionen und den Basisvektoren des Fourierraums aufgefasst werden.

Die Fourier-Transformation ist prädestiniert, globale Aussagen über ein Signal f(t) zu treffen, da sich ihre Basisfunktionen über den gesamten Zeitbereich erstrecken und keine zeitliche Lokalisierbarkeit zulassen.

Zur Analyse von transienten oder zeitlich begrenzten Signalen wurde die Kurzzeitfouriertransformation (STFT) entwickelt, die eine Entwicklung einer eindimensionalen Zeitfunktionen in die zweidimensionale Zeit-Frequenz-Ebene entspricht. Dabei wird das Signal in als quasistationär angenommene Bereiche aufgeteilt und diese werden unabhängig voneinander einer gefensterten Fourier-Transformation unterzogen (Jürgen Niederholz, Anwendungen der Wavelet-Transformation in Übertragungssystemen, Universität Duisburg, 1999 (http://www.ub.uni-duisburg.de/diss/diss0016/inhalt.htm). Die Fensterfunktionen im Zeitbereich bewirkt ebenfalls eine Fensterung im Frequenzbereich, so dass die STFT über den ganzen Frequenzbereich eine konstante effektive Bandbreite $\Delta\omega$ und über den gesamten Zeitbereich eine effektive zeitliche Ausdehnung $\Delta t$ besitzt.

Die konstante effektive Bandbreite und die Beschränkung auf Sinus- und Kosinusfunktionen wurde bei der STFT immer noch als Einschränkung empfunden. Beide Beschränkungen werden bei der Wavelettransformation aufgehoben. Eine allgemeine Einführung in die Wavelettransformation findet sich in (Antje Ohlhoff, Anwendung der Wavelettransformation in der Signalverarbeitung, Universität Bremen, 1996). Die Funktion $\Psi(t)$ stellt ein gültiges Wavelet dar, wenn eine reelle, endliche Konstante $C_\Psi$ existiert, wobei $\tilde{\Psi}(\omega)$ die Fouriertransformierte der Funktionen $\Psi(t)$ ist.

$$C_\Psi = \int\limits_{-\infty}^{\infty} \frac{\left|\tilde{\Psi}(\omega)\right|^2}{\omega} d\omega \tag{12}$$

Eine wesentliche Idee der Wavelettransformation besteht darin, die Funktion $\Psi(t)$ schon geschickt zu wählen und dem zu lösenden Problem anzupassen, so dass die zu analysierenden Funktionen mit einer geringen Anzahl von Wavelets beschrieben werden können. Anders ausgedrückt soll sich nur eine geringe Anzahl von Koeffizienten der Basisfunktionen nennenswert von 0 unterscheiden.

Ausgehend von einem Basiswavelet oder Motherwavelet werden die übrigen Basisfunktionen, die eine vollständige Basis für den Funktionsraum bilden, mit einem Translationsparameter b und einem Dilatationsparameter a gemäß Gleichung (13) erzeugt.

$$\Psi_{a,b}(t) = \frac{1}{\sqrt{|a|}} \Psi\left(\frac{t-b}{a}\right); a, b \in \Re, a\neq 0 \tag{13}$$

Durch die Gleichung (13) wird die so genannte Güte Q = $\omega_M/\Delta\omega$ über den Frequenzbereich konstant gehalten, der im Kontext der Wavelettransformation auch als Skalierungsbereich bezeichnet wird. Hierbei ist $\omega_M$ eine Mittenfrequenz. In der Natur treten viele Phänomene auf, bei denen die Güte konstant ist.

Die GB 2 275 108 A offenbart ein Flussmessgerät für Fluide. Der mechanische Aufbau ähnelt der in Figur 12 dargestellten Bauform. Ein Signalgenerator erzeugt mindestens zwei feste Frequenzen. Die Abtastfrequenz des empfangenen Signals in einem Analog-Digital-Wandler ist beispielsweise das 32-fache der Ultraschallfrequenz. Ein Prozessor bestimmt den Fluidfluss. Um das abgetastete, empfangene Signal mit einem Referenzsignal zu korrelieren, werden die Abtastwerte sowohl mit einer Sinus- wie auch mit einer Kosinus-Funktion multipliziert und die Produkte mit der Sinus- und Kosinus-Funktion aufsummiert, so dass man eine Sinusproduktsumme für die Produkte mit der Sinus-Funktion und eine Kosinusproduktsumme für die Produkte mit der Kosinus-Funktion erhält. Die Phase ergibt sich als Arcustangens des Quotienten aus Sinusproduktsumme geteilt durch Kosinusproduktsumme.

Die US 2002/0143479 A1 und die EP 0 797 105 A2 enthalten ähnliche Offenbarungen.

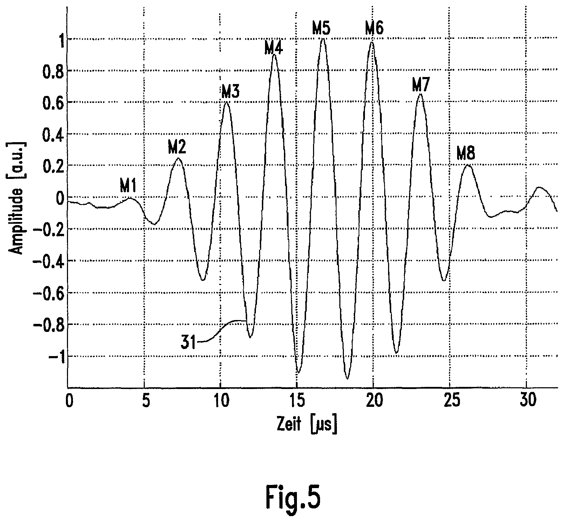

Auch die US 6,305,233 B1 beschäftigt sich mit der digitalen Bestimmung von Geschwindigkeiten mittels Ultraschallflussmessungen. Der mechanische Aufbau ähnelt ebenfalls der in Figur 12 dargestellten Bauform. Wie üblich wird das von einem Ultraschallwandler empfangene analoge Signal digitalisiert, wodurch Digitalsignaldaten erhalten werden. Aus den Digitalsignaldaten wird ein Messteil ausgewählt. Aus dem Messteil wird eine Vielzahl von Steigungen $S_i$ für je ein Messsegment bestimmt, wobei i eine ganze Zahl zwischen 0 und 20 ist. Jedes der Messsegmente liegt um einen Signalniveauwert V herum, der durch Mitteln der Digitalsignaldaten bestimmt wird, die vor dem Messteil empfangen werden. Der Digitalniveauwert V stellt also eine Art Arbeitspunkt, Mittelwert oder Gleichanteil der Digitalsignaldaten dar. Der Digitalniveauwert V ist in Figur 5 dieser Schrift etwa die mittlere Linie zwischen der oberen, mit TOP REF. und FF bezeichneten Linie und der unteren, mit BOTTOM REF. und 00 bezeichneten Linie. Zur Bestimmung der Steigungen $S_i$ wird in jedem Meßsegment i eine Anzahl von k Punkten aus den Digitalsignaldaten ausgewählt, die um den Digitalniveauwert V herum liegen. An die ausgewählten k Punkte in jedem der Messsegmente wird eine gerade Linie gefittet, von der die Steigung $S_i$ bestimmt wird. Aus den Steigungen Si wird dann eine Ankunftszeit t des akustischen Wellenpakets bestimmt. Hierzu wird für ausgewählte Messsegmente eine entsprechende Messzeit $A_i$ bestimmt, die angibt, wann das analoge Signal den Digitalniveauwert V schneidet. Jeder Messzeit $A_i$ wird ein Gewichtungsfaktor $W_i$ zugeordnet. Zur Bestimmung der Ankunftszeit t des akustischen Wellenpakets wird noch die Produktsumme aus Messzeiten $A_i$ und Gewichtungsfaktoren $W_i$ bestimmt.

Es ist Aufgabe der Erfindung ein Flussmessgerät und ein Verfahren zur Bestimmung der zeitlichen Lage eines Wellenpakets anzugeben, die höhere Flüsse messen können.

Diese Aufgabe wird durch die Lehre der unabhängigen Ansprüche gelöst.

Bevorzugte Ausführungsformen der Erfindung sind Gegenstand der Unteransprüche.

Vorteilhaft an der Verwendung einer gewichteten Summe von Messwerten eines Wellenpakets zur Bestimmung der zeitlichen Lage des Wellenpakets ist, dass hierdurch ein großer Teil der Energie des Wellenpakets ausgewertet wird, womit die zeitliche Unschärfe klein wird und somit die zeitliche Lage sehr genau bestimmt werden kann.

Vorteilhafterweise wird zum Gewichten eine Vergleichsfunktion verwendet, die der Form des Wellenpakets ähnelt. Eine Sinus- und Kosinusfunktion ähnelt zumindest einer Periode oder wenigen Perioden des Ultraschallpakets.

Die Verwendung einer Sinus- und einer Kosinusfunktion als Vergleichsfunktionen ist vorteilhaft, weil hierdurch die Lage des Wellenpakets innerhalb einer Periode mit Hilfe einer Arcustangensfunktion bestimmt werden kann, ohne weitere Vergleichsfunktionen zu berechnen.

Die Verwendung einer exponentiellen Hüllkurve, die einer Gauß'schen Glockenkurve ähnelt, passt die Gesamtform der Vergleichsfunktion an die Form eines Wellenpakets an. So ist es möglich, praktisch die gesamte Energie des Wellenpakets auszuwerten und die zeitliche Lage mit einer optimalen Genauigkeit zu bestimmen.

Die iterative Berechnung der zeitlichen Lage eines Wellenpakets reduziert die Anzahl der notwendigen Berechnungen von Produktsummen und kann so Rechenzeit sparen.

Um zu prüfen, ob die zeitliche Lage des Wellenpakets nicht um eine oder mehrere Perioden verschoben ist, können die Vergleichsfunktionen um eine oder mehrere Perioden verschoben werden und an diesen Stellen Produktsummen berechnet werden. Zu diesem Zweck kann auch die geometrische Summe zweier Summenprodukten berechnet werden, was mit einem noch geringeren Rechenaufwand verbunden ist. Zum gleichen Zweck können auch die Messwerte direkt mit Bruchteilen von Maxima und/oder Minima verglichen werden.

Eine Maximumsuche in Produktsummen von Messwerten mit einer unterschiedlich weit verschobenen Vergleichsfunktion erlaubt es, den Programmcode kurz zu halten.

Eine quadratische Interpolation mittels einer Parabel ist gut geeignet zur Maximumsuche. Außerdem stimmt der Verlauf einer Parabel in der Nähe des Scheitels gut mit dem Verlauf eine Sinus- oder Kosinusfunktion bei einem Maximum überein, da die Taylorentwicklung zweiter Ordnung hier ausreichend ist.

Im Folgenden werden bevorzugte Ausführungsformen der Erfindung unter Bezugnahme auf die beiliegenden Zeichnungen näher erläutert. Dabei zeigen:

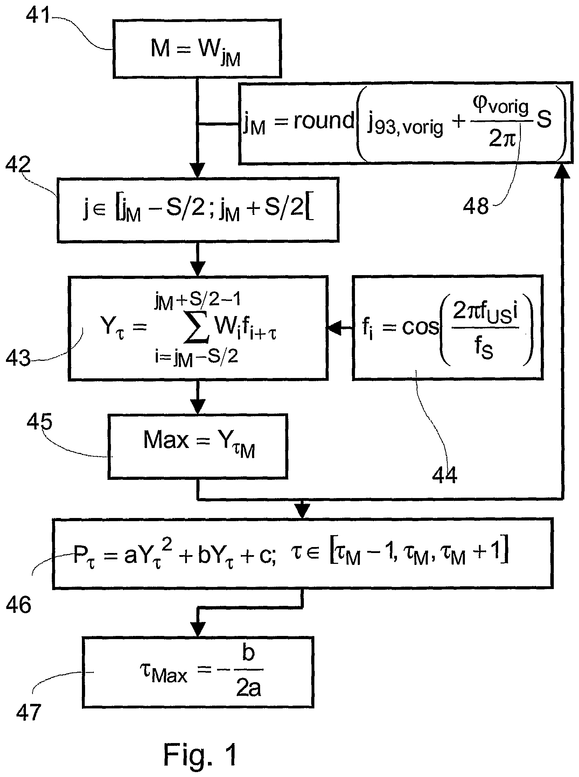

Fig. 1 ein Flussdiagramm eines TiDO Algorithmus;

Fig. 2 ein Flussdiagramm eines TriTraX Algorithmus;

Fig. 3 ein Flussdiagramm eines Teiles eines WaveTraX Algorithmus;

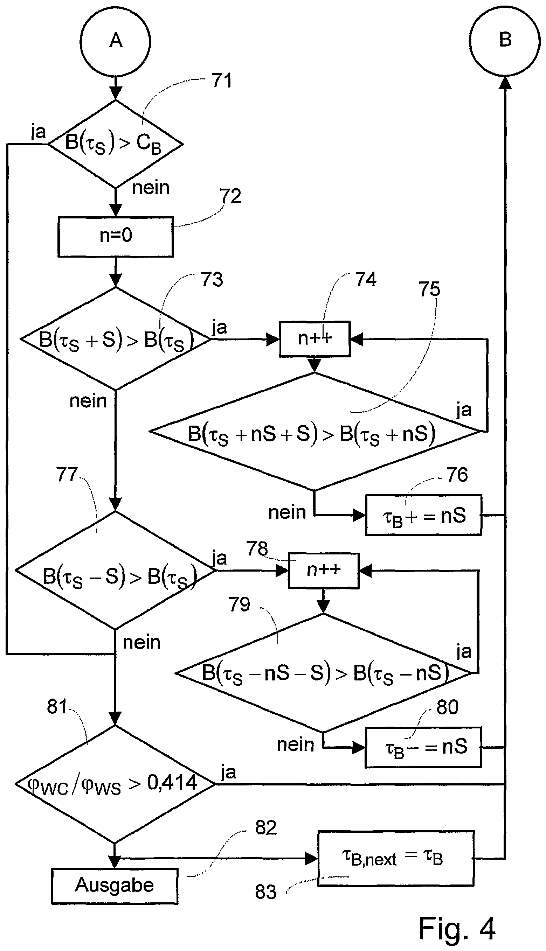

Fig. 4 ein Flussdiagramm eines zweiten Teiles des WaveTraX Algorithmus;

Fig. 5 ein typisches empfangenes Ultraschallpaket beim TiDO, TriTraX oder WaveTraX Algorithmus;

Fig. 6 die Anregungen beim P-SEX Algorithmus;

Fig. 7 ein typisches empfangenes Ultraschallpaket beim P-SEX Algorithmus;

Fig. 8 eine Referenzfunktion beim P-SEX Algorithmus;

Fig. 9 eine Kreuzkorrelationsfunktion zwischen einem Ultraschallpaket und einer Referenzfunktion beim P-SEX Algorithmus;

Fig. 10 eine Bauform eines bekannten Spirometers mit einer erfindungsgemäßen Beschaltung; und

Fig. 11 eine Bauform eines bekannten Spirometers; und

Fig. 12 eine weitere Bauform eines bekannten Spirometers.

Wie sich aus obigen Formeln, insbesondere Formel (8) ergibt, muss insbesondere der Laufzeitunterschied $\Delta t$ und die mittlere Laufzeit $t_0$ möglichst exakt gemessen werden. Die anderen Größen, die in den Volumenstrom V eingehen, also $C_1$, $C_2$, L, $\alpha$, und/oder r, sind weitgehend konstant und können entweder direkt oder über Kalibrationsmessungen bestimmt werden. Auch die Nichtlinearitäten, die dazu führen, dass $C_1$ und $C_2$ von V und damit von $\Delta t$ abhängen, kann man mit Eichmessungen und Anpassung von Modellkurven wie beispielsweise Polynomen an die Messwerte in den Griff bekommen.

Es verbleibt also das technische Problem, den Laufzeitunterschied $\Delta t$ oder die Laufzeiten $t_{45}$ und $t_{54}$ ausreichend genau zu messen. Dieses Problem ist äquivalent dazu, die zeitliche Lage von Ultraschallpaketen zu bestimmen, die ja Anfang und Ende von $\Delta t$ festlegen.

Bei den hier beschriebenen Ausführungsformen werden Ultraschallwandler SC.131.XLV (SECO Sensor Consult GmbH) eingesetzt. Für diese Ultraschallwandler wird eine typische Mittenfrequenz von 310kHz, eine typische Bandbreite ($\pm$3dB) von $\pm$40kHz sowie eine Schallkeulenbreite von 11° (-3dB) angegeben. Es wird Ultraschall der Frequenz $f_{US}$=312,5kHz verwendet, weil es vorteilhaft aber nicht notwendig ist, wenn die Abtastrate $f_s$ ein ganzzahliges Vielfaches der Ultraschallfrequenz $f_{US}$ ist Ultraschall unterliegt bei Normalbedingungen praktisch keiner Dispersion. Für die angestrebte Genauigkeit der Flussmessung muss die zeitliche Lage eines Ultraschallpakets auf etwa eine Nanosekunde genau bestimmt werden. Es ist mit hohen Kosten verbunden, das von einem Ultraschallwandler gelieferte Signal mit einer Abtastrate von einem Gigahertz abtasten und dann das Maximum in den Abtastwerten suchen. Trotzdem wird diese Ausführungsform als eine erfindungsgemäße Ausführungsform betrachtet.

Es hat sich gezeigt, dass die im folgenden beschriebenen Algorithmen die geforderte zeitliche Genauigkeit von 1 ns bereits bei Abtastraten $f_S$ = 5 MHz liefern. Mit moderatem technischen und finanziellen Aufwand sind derzeit Abtastraten bis 25 MHz erreichbar.

Das vom Analog-Digital-Wandler 94 oder 95 gelieferte Signal enthält Messwerte $W_j$ die einen zeitlichen Abstand von $1/f_S$ aufweisen. Ein typisches Ultraschallpaket ist in Fig. 5 gezeigt. Die lokalen Maxima sind mit M1 bis M8 durchnummeriert. M5 markiert das globale Maximum.

Die Messwerte $W_j$ sind rauschbehaftet und weisen einen unbekannten Offset auf. Deshalb liefert die Suche nach lokalen Maxima oder Nullstellen ohne vorherige Signalaufbereitung keine brauchbaren Ergebnisse, selbst wenn die Abtastrate ausreichend hoch wäre.

**TiDO Algorithmus**

Der Time Domain Only (TiDO) Algorithmus beruht im wesentlichen auf einer Bestimmung der Phasenlage einer einzelnen Schwingungsperiode im Wellenpaket. Die meiste Energie und damit den größten Rauschabstand (SNR, signal to noise ratio) weist die Schwingung mit der größten Amplitude auf, wobei das additive Rauschen als konstant über das gesamte Wellenpaket angenommen wird. Die Laufzeit wird in mehreren Schritten bestimmt:

1. Im ersten Schritt 41 wird das absolute Maximum M = $W_{jM}$ an der Stelle $j_M$ bestimmt. Der erste Schritt wird nur dann durchgeführt, wenn beispielsweise nach dem Einschalten des Geräts kein vorausgehender Messwert $\tau_{M,vorig}$

zur Verfügung steht. Falls ein vorausgehender Messwert zur Verfügung steht, kann der Schritt 41 durch den Schritt 48 ersetzt werden, der weiter unten beschrieben wird.

Die maximal zu erwartete Änderung der Laufzeiten $t_{45}$ und $t_{54}$ liegt im Bereich von $\pm 20\mu s$, wobei die Laufzeiten etwa $160\mu s$ betragen. Einschließlich der Länge des Schallpakets von etwa $30\mu s$ müssen also in Schritt 41 etwa $70\mu s \cdot f_S$ Datenpunkte $W_i$ analysiert werden.

Unter Berücksichtigung der Periodizität des Schallpakets kann der Suchalgorithmus in Schritt 41 optimiert werden, so dass nicht alle Messpunkte überprüft werden müssen. Man beginnt bei einem Schätzwert für die Stelle des globalen Maximums $j_M$. Dies kann ähnlich zu Schritt 48 das in der vorausgehenden Messung bestimmte Maximum $\tau_{M,vorig}$ oder nach dem Einschalten des Geräts auch eine beliebige Stelle sein. Man initialisiert M mit $W_{\tau M,vorig}$. Man prüft die Stelle $j_{M+1}$ rechts davon. Falls $Wj_M < W_{jM+1}$ geht man in dieser Richtung weiter, bis $W_{jE}$ < M * 0,99. Dabei wird das neue Maximum M gespeichert. Falls jetzt $W_{jM} > 0,99M$ prüft man noch die andere Richtung nach links bis $W_{jA} < 0,99M$. Falls es ein neues Maximum gibt, wird das in M gespeichert. Als Zwischenergebnis erhält man so ein lokales Maximum $M = W_{jM}$. Im ungünstigsten Fall wurden bis jetzt etwas mehr als S/2 Stellen verglichen. Jetzt prüft man $W_{jM-nS}$ und $W_{jM+nS}$, n = 1, 2, .... Das Maximum dieser Werte ist ein ausreichend guter Schätzwert für das absolute Maximum. Aufgrund der glockenförmigen Hüllkurve stellt der Schätzwert nicht notwendigerweise das Maximum dar, und es ist zu erwarten, dass der Schätzwert um so schlechter ist, je höher n ist.

2. Die Datenpunkte $W_j$ im Intervall

$$j \in \left[ j_M - S/2 \, ; \, j_M + S/2 \right[ \tag{14}$$

entsprechen nun der Schwingungsperiode mit der größten Amplitude, die mittels einer rechteckförmigen Fensterfunktionen in Schritt 42 zur weiteren Signalverarbeitung "ausgeschnitten" wird. Dabei ist S die Anzahl der Messwerte in einer Schwingungsperiode des Ultraschallpakets, wobei

$$S = f_S / f_{US} \tag{15}$$

Für eine Abtastfrequenz $f_S$ von 5MHz und fus von 312,5kHz hat S einen Wert von 16.

3. Nun wird die zeitdiskrete zyklische Variante $Y_\tau$ der Kreuzkorrelationsfunktion in Schritt 43 berechnet:

$$Y_\tau = \sum_{i=j_M-S/2}^{j_M+S/2-1} W_i f_{i,\tau} \tag{16}$$

$$f_i = \cos\left( \frac{2\pi f_{US} i}{f_S} \right), \quad -\pi <= \frac{2\pi f_{US} i}{f_S} < \pi \tag{17}$$

Die Berechnung von Gleichung (17) erfolgt in Schritt 44. Die Werte $f_i$ können offline, zum Beispiel während der Kompilierung oder Assemblierung berechnet werden und in einem Feld, das auch als Lookup-Tabelle bezeichnet wird, abgelegt werden. Aus diesem Feld werden die Werte $f_i$ zur Laufzeit ausgelesen. Bei Verwendung von Formeln (16) und (17) müssen in der Lookup-Tabelle etwa zwei Perioden das Kosinus gespeichert werden. Durch explizite Rückführung auf die Grundperiode gemäß Gleichung (17a) kann die Lookup-Tabelle auf eine Periode beschränkt werden.

$$f_{i,\tau} = \begin{cases} f_{i+\tau} & \forall \quad i+\tau \le S \\ f_{i+\tau-S} & \forall \quad i+\tau > S \end{cases} \tag{17a}$$

4. In $Y_\tau$ wird nur das Maximum an der Stelle $\tau_M$ in Schritt 45 gesucht. Es ist zu erwarten, dass $\tau_M \approx j_M$ ist. Eine leichte Verschiebung kann sich durch Rauschen in den Messpunkten $W_j$ ergeben. Die Schrittweite von $Y_\tau$ entspricht der von $W_j$ und damit $1/f_S$. Sie genügt damit noch nicht der erforderlichen Genauigkeit von 1 ns.

5. Eine Möglichkeit zur genaueren Maximumsbestimmung ist, eine Parabel $P_\tau$ gemäß Formel (18) in Schritt 46 durch das Maximum $Y_{\tau M}$ und die beiden Nachbarpunkte $Y_{\tau M-1}$ und $Y_{\tau M-1}$ zu legen und die Position des Scheitelpunkts $\tau_{Max}$ gemäß Formel (19) in Schritt 47 zu bestimmen.

$$P_\tau = aY_\tau^2 + bY_\tau + c \tag{18}$$

$$\tau_{Max} = -\frac{b}{2a} = \frac{1}{2f_S}\frac{Y_{\tau M+1} - Y_{\tau M-1}}{Y_{\tau M-1} + Y_{\tau M+1} - 2Y_{\tau M}} \tag{19}$$

Die zeitliche Lage des Ultraschallpakets ergibt sich nun aus Gleichung (20):

$$t = \frac{\tau_{Max}}{f_S} \tag{20}$$

Der TiDO Algorithmus würde bereits eine ausreichende Genauigkeit liefern. Es ist jedoch nötig, viele Datenpunkte für den ersten Schritt 41 zu analysieren, da sichergestellt werden muss, dass, auch bei maximaler Änderungsrate der Schalllaufzeit vor allem auf Grund einer Änderung der Strömungsgeschwindigkeit v des Mediums, sich das in Schritt 42 ausgewählte Intervall in den zu analysierenden Messpunkten befindet. Deshalb wird noch Schritt 48 eingeführt:

6. Das in der vorausgehenden Messung bestimmte Maximum $\tau_{M,vorig}$ wird in der nachfolgenden Messung als das im ersten Schritt 41 gefundene Maximum $j_M$ angenommen (Schritt 48), wodurch die Maximumsuche in Schritt 41 überflüssig wird. Es ist dabei nicht wichtig, dass das Maximum $M = W_{jM}$ in der Mitte des in Schritt 42 ausgewählten Intervalls liegt. Auch die beiden Nachbarperioden enthalten nicht viel weniger Energie als die Periode mit dem Maximum. Wichtig ist jedoch, dass das Maximum in dem in Schritt 42 ausgewählten Intervall liegt. Dies ist der Fall, wenn sich die Phase zwischen zwei Wiederholungen der Schritte 42 bis 49 nicht um mehr als eine halbe Periode ändert. Da beim Prototyp Messraten von 1 kHz erreicht wurden und aus den Laufzeiten von etwa 160μs eine maximale Messrate von 6,25kHz abgeleitet werden kann, stellt dies keine wirkliche Einschränkung dar.

Diese Überlegungen gelten jedoch nur für kleine Flüsse, bei denen Wirbelzonen ausreichend klein sind. An der oberen Messgrenze von 20l/s werden bei einem Rohrdurchmesser von etwa 2 cm Geschwindigkeiten von 65 m/s oder 240 km/h erreicht. Bei hohen Flüssen wurde beobachtet, dass die Laufzeit zwischen zwei aufeinander folgenden Messungen so stark variiert, dass sich die Referenzstelle - also $j_M$ beim TiDO Algorithmus oder $j_{93}$ beim TriTraX Algorithmus - zwischen zwei aufeinander folgenden Messungen um mehr als eine Ultraschallperiode $\approx 3\mu s$ unterscheidet. Dies scheint auf Wirbel zurückzuführen zu sein. Diese Wirbel führen zusammen mit der Signalverwehung zu einer um 20dB kleineren Amplitude des Empfangssignals bei einem Fluss von 20 l/s.

Aufgrund der Variation der Laufzeit bei hohen Flüssen kann es sinnvoll sein, in Schritt 45 das Maximum $Y_{\tau M}$ mit den benachbarten Maxima $Y_{\tau M\pm nS}$, n=1,2, ... oder $W_{\tau M}$ mit $W_{\tau M\pm nS}$ zu vergleichen. Sollte sich hierbei herausstellen, dass $Y_{\tau M\pm nS}$ bzw. $W_{\tau M\pm nS}$ das absolute Maximum ist, sollten die Schritte 42 bis 45 mit $\tau_M\pm nS$ als Schätzwert für $j_M$ wiederholt werden.

Aufgrund der Variation der Laufzeit bei hohen Flüssen kann es bei allen in dieser Schrift beschriebenen Ausführungsformen ferner sinnvoll sein, die Laufzeiten aus mehreren aufeinander folgenden Messungen zu mitteln. Die Anzahl der gemittelten Messungen kann mit dem Fluss zunehmen.

Nachteilig ist, dass sich aufgrund des Temperaturverhaltens und der Flussabhängigkeit der Richtcharakteristik der Ultraschallwandler die in Fig. 5 dargestellte Wellenform des Ultraschallpakets ändern kann. Deshalb, aufgrund von Rauschen, und des geringen Höhenunterschieds von 1% zwischen den Maxima M5 und M6 kann das Maximum M6 zum absoluten Maximum werden. Bei starker Verwirbelung kann sogar M4 oder M7 zum globalen Maximum anwachsen. Deshalb erweist sich der TiDO Algorithmus als wenig robust und praxistauglich.

Nachteilig ist ferner, dass die Maxima der angefitteten Kosinusfunktion $f_i$ und der Parabel $P_\tau$ umso weiter

auseinander liegen, je geringer die Abtastrate $f_S$ ist.

**TriTraX Algorithmus**

Diese beiden Probleme soll der Trigonometric Transform by Cross **(X)** Correlation (TriTraX) Algorithmus lösen. Deshalb wird ein Punkt im Wellenpaket benutzt, der die Schwingungsperiode innerhalb des Ultraschallpakets zuverlässiger identifiziert. In Fig. 5 beträgt der Höhenunterschied zwischen den Maxima M4 und M5 etwa 14% der Höhe von M5 und ist zuverlässig detektierbar.

1. Im ersten Schritt 51 wird wieder das absolute Maximum M = $W_{jM}$ des Ultraschallpakets gesucht.
Aufgrund der Variation der Laufzeit bei hohen Flüssen kann es hierbei sinnvoll sein, in Schritt 51 das Maximum Wjm mit den benachbarten Maxima $W_{\tau M \pm nS}$, n=1,2, ... zu vergleichen. Sollte sich hierbei herausstellen, dass $W_{\tau M \pm nS}$ das absolute Maximum ist, wird $_{jM=jM \pm nS}$ gesetzt.
2. Nun wird der Punkt $j_{93}$ in bestimmt, dessen Ordinatenwert $W_{j93}$ am nächsten bei 0,93*M liegt oder diesen Wert zum ersten Mal in einem Wellenpaket überschreitet. Zur Optimierung des Signalrauschabstandes (SNR) wird die Periode mit der größten Amplitude für die weiteren Schritte im Algorithmus in Schritt 52 ausgewählt:

$$j \in \left[ j_{93} - \frac{3}{8}S; \ j_{93} + \frac{5}{8}S \right[ \tag{21}$$

$j_{93}$ liegt etwa 1/8 Periode vor M5. Für die exakte Bestimmung der exakten, also interpolierten Position Maximums M5 ist es nicht wichtig, dass die ausgewählte Periode exakt von Minimum zu Minimum verläuft.
In anderen Ausführungsformen kann jedoch auch ein anderer Ordinatenwert als 93% des Maximums gewählt werden. Insbesondere kann dieser Ordinatenwert auch auf das Minimum, das arithmetische Mittel von Maximum und Minimum, die Differenz zwischen Maximum und Minimum und/oder den Gleichanteil des Wellenpakets bezogen sein. Ist der Ordinatenwert kleiner als der Gleichanteil des Wellenpakets, ist es besser, ein erstmaliges Unterschreiten des Ordinatenwerts als Bezugspunkt zu wählen.
3. Nun werden die Korrelationskoeffizienten $\varphi_C$ und $\varphi_S$ zwischen $c_j$ und $W_j$ bzw. $s_j$ und $W_j$ in Schritten 54 und 55 berechnet, wobei

$$c_j = \cos\left( \frac{2\pi f_{US} j}{f_S} \right) \tag{22}$$

$$s_j = \sin\left( \frac{2\pi f_{US} j}{f_S} \right) \tag{23}$$

Die Formeln (22) und (23) werden in Schritten 53 und 56 berechnet. Dies kann offline erfolgen, sodass die Werte während des Betriebs aus Feldern (Lookup-Tabellen) gelesen werden.
Die Berechnung der Korrelationskoeffizienten entspricht mathematisch der Bestimmung eines komplexen Koeffizienten $\varphi_C + i\varphi_S$ einer diskreten Fourierentwicklung:

$$y_j = \left( \varphi_C + i\varphi_S \right) \exp\left( i \frac{2\pi f_{US} j}{f_S} \right) \tag{24}$$

4. An Stelle dieser komplexen Darstellung kann auch der Betrag und die Phase $\varphi$ der Schwingung angegeben werden, wobei die Phase $\varphi$ mittels der arctan-Funktion berechnet werden kann. Diese ist jedoch nur in einem Intervall der Länge $\pi$ eindeutig. Durch Berücksichtigung der Vorzeichen von $\varphi_C$ und $\varphi_S$ kann dieses Intervall auf $2\pi$ ausgedehnt werden:

$$\varphi = \arctan 2(\varphi_S ; \varphi_C) \tag{25}$$

In entsprechender Weise ist arc cot2($\varphi_C;\varphi_S$) definiert.

Die Phase $\varphi$ wird in Schritt 57 berechnet.

5. Die Laufzeit wird aus Formel 26 berechnet:

$$t = \left( j_{93} + \frac{1}{8} + \frac{\varphi}{2\pi} S \right) \frac{1}{f_S} \tag{26}$$

6. Analog zum oben beschriebenen TiDO Algorithmus wird wieder die Annahme in Schritt 59 getroffen, dass

$$j_M = \mathrm{round}\left( j_{93,\mathrm{vorig}} + \frac{\varphi_{\mathrm{vorig}}}{2\pi} S \right) \text{ die Stelle des absoluten Maximums } M = W_{j_M} \text{ ist, um Schritt 51 aus Gründen}$$

der Rechengeschwindigkeit zu umgehen.

Wie beim TiDO Algorithmus kann zunächst ein lokales Maximum gesucht werden und dann im Abstand von $\pm nS$ benachbarte lokale Maxima überprüft werden, um das globale Maximum zu finden, wobei n eine ganze Zahl ist.

Sowohl der TiDO als auch der TriTraX Algorithmus haben den Nachteil, dass nur eine Periode des gesamten Ultraschallpakets ausgewertet wird, was zu einer sub-optimalen Genauigkeit der Bestimmung der Laufzeit führt.

## WaveTraX Algorithmus

Der **Wave**let **Tra**nsform by Cross **(X)** Correlation Algorithmus ermöglicht es, eine dem Signal angepasste Basis zu verwenden. Das Motherwavelet wird so gewählt, dass es möglichst genau einem Ultraschallpaket entspricht.

Aus einem nichtlinearen Fit über ein typisches Ultraschallpaket ergab sich das Mothersinus- und - cosinuswavelet zu:

$$S_j = \sin\left( \frac{2\pi f_{US} j}{f_S} \right) \cdot \exp\left( -\left| \frac{j}{8{,}6 \cdot 10^{-6}\,s \cdot f_S} - 1{,}957 \right|^{3,24} \right), \text{ wobei } 0 \le j \le 10S, \; n \in N \tag{27}$$

$$C_j = \cos\left( \frac{2\pi f_{US} j}{f_S} \right) \cdot \exp\left( -\left| \frac{j}{8{,}6 \cdot 10^{-6}\,s \cdot f_S} - 1{,}957 \right|^{3,24} \right), \text{ wobei } 0 \le j \le 10S, \; n \in N \tag{28}$$

Die Motherwavelets werden in den Schritten 61 und 62 berechnet. Die Ergebnisse können in Feldern abgelegt sein.

1. Zunächst wird in Schritt 63 die Lage des Wellenpaket mit Hilfe einer Kreuzkorrelationsfunktionen aus den Messwerten $W_j$ mit dem Mothersinuswavelet ermittelt.

$$K_{WS,\tau} = \sum_{j=0}^{10S} S_j W_{j+\tau} \tag{29}$$

Die Kreuzkorrelationsfunktion $K_{WS,\tau}$ Erreicht ihren Maximalwert, wenn die Lage des Mothersinuswavelets und des abgetasteten Ultraschallpakets möglichst guter Übereinstimmung ist. Eine Maximumsuche in $K_{WS,\tau}$ liefert die Stelle $\tau_M$ des Maximums der Kreuzkorrelationsfunktion. Das Maximum der Kreuzkorrelationsfunktion ist gleichzeitig der Korrelationsfaktor $\varphi_{WS}$ zwischen den Messwerten und dem Mothersinuswavelet:

$$\varphi_{WS} = K_{WS,\tau_M} \tag{30}$$

In diesem Zusammenhang soll schon jetzt darauf hingewiesen werden, dass in einer verfeinerten Ausführungsform in die Maximumsuche eine Iteration eingebaut werden kann, gemäß der in Gleichung (30) bei einem beliebigen $\tau$, also nicht unbedingt bei $\tau_M$ aber beispielsweise bei $\tau_{M,vorig}$, dem $\tau_M$ der vorangehenden Messung, begonnen wird. Aus Gleichung (34) wird dann in Schritt 66 ein verbesserter Schätzwert $\tau_B$ für $\tau_M$ ermittelt. Ergebnis dieser Iteration ist ein lokales Maximum. Die Maximumsuche in den Schritten 72 bis 80 beschränkt sich dann auf lokale Maxima, deren Abstand zum gerade bestimmten Maximum sich im Wesentlichen aus der Frequenz des Ultraschalls ergibt.

2. Die exakte Phasenlage des Wellenpaket wird ähnlich dem TriTraX Algorithmus aus den Korrelationsfaktoren zwischen den Messwerten und dem Mothersinus- und -cosinuswavelet in Schritt 65 ermittelt. Dies ist äquivalent zur Ermittlung der Phasenlage aus der Phase eines komplexen Korrelationsfaktors. Der Korrelationsfaktor zwischen den Messwerten und dem Motherkosinuswavelet wird aus

$$\varphi_{WC} = \sum_{j=0}^{10S} C_j W_{j+\tau_M} \tag{31}$$

in Schritt 64 bestimmt.

3. Aus den Korrelationsfaktoren wird die Phase $\varphi_W$ bestimmt:

$$\varphi_W = \arccot 2\left(\varphi_{WC}; \varphi_{WS}\right) \tag{32}$$

Die Laufzeit ergibt sich aus:

$$t = \left(\tau_M + \frac{\varphi_W}{2\pi} S\right) \frac{1}{f_S} \tag{33}$$

Steht bereits ein Schätzwert $\tau_S$ für den Index $\tau_M$ des Maximums zur Verfügung, so kann für die gleichen Messwerte $W_j$ ein verbesserter Schätzwert $\tau_B$ aus

$$\tau_B = \tau_S + \frac{\varphi_W}{2\pi} \tag{34}$$

in Schritt 66 berechnet werden. $\varphi_W$ ergibt sich aus Gleichung (32), wobei $\varphi_{WS}$ und $\varphi_{WC}$ aus Gleichungen (35) und (36) in Schritten 63 und 64 berechnet werden.

$$\varphi_{WS} = \sum_{j=0}^{10S} S_j W_{j+\tau_S} \tag{35}$$

$$\varphi_{WC} = \sum_{j=0}^{10S} C_j W_{j+\tau_S} \tag{36}$$

$\tau_B$ gibt nicht notwendigerweise exakt die Phasenlage des Ultraschallpakets an, weil die Exponentialfunktion in den beiden Motherwavelets eine Hüllkurve für die Sinus- bzw. Kosinus-Anteile darstellt und diese verzerrt. Stimmt die Hüllkurve des empfangenen Ultraschallpakets (vgl. Fig. 5) noch nicht mit dem Exponentialanteil der beiden Motherwavelets überein, weil der Abstand vom globalen Maximum $|\tau_S - \tau_M|$ noch zu groß ist, ist nicht zu erwarten, dass der verbesserte Schätzwert $\tau_B$ exakt die Phasenlage des Ultraschallpakets angibt. Insofern kann der Betrag von Korrelationsfaktor $\varphi_{WC}$ oder $\varphi_W$ in Schritt 81 als Maß für die Konvergenz herangezogen werden.

Gemäß Gleichung (29) wird das Maximum des Korrelationsfaktors $\varphi_{WS}$ mit dem Mothersinuswavelet (vgl. Gleichung (30)) bestimmt. Aufgrund der begrenzten Abtastrate weicht dieses Maximum optimalerweise um bis zu $\pm 1/(2f_S)$ von der Phasenlage des Ultraschallpakets ab. Die tatsächlichen Abweichungen sind noch etwas größer. Die Nachkommastellen vom Index $\tau_M$, der ja zunächst ganzzahlig ist, werden sozusagen mit Hilfe des Motherkosinuswavelets berechnet. Solange das Motherkosinuswavelet noch eine Verschiebung liefert, deren Betrag größer als ein Schwellenwert ist, kann man davon ausgehen, dass noch keine ausreichende Konvergenz erreicht wurde. Eine sinnvolle Wahl dieses Schwellenwerts ist $1/f_S$. Für S=16 ergibt sich eine betragsmäßige Obergrenze für $\varphi_W$ von $\pi/8$ oder 22,5° und damit

$$\varphi_{WC}/\varphi_{WS} > 0{,}414 \tag{37}$$

Diese Obergrenze kann für den optimalen Fall für $\varphi_W$ auf 11,25° und für $\varphi_{WC}/\varphi_{WS}$ auf 0,199 abgesenkt werden.

Falls diese Obergrenze überschritten wird und deshalb ein weiterer Iterationsschritt durchgeführt werden soll, wird $\tau_B = \tau_M$ zwischen den beiden Iterationsschritten in Schritt 68 auf einen ganzzahligen Wert gerundet. Mit $\tau_M$ wurde der interpolierten Index des Maximums von $\varphi_{WC}$ bezeichnet. Gegen diesen konvergiert $\tau_B$. Andernfalls müssten die Messwerte $W_j$ interpoliert und die Funktionswerte der beiden Motherwavelets $S_j$ und $C_j$ neu berechnet werden. Insofern ist $\tau_S$ in Gleichung (34) ganzzahlig, wohingegen $\tau_B$ typischerweise Nachkommastellen aufweist.

In einer Ausführungsform, in der ein Mikroprozessor verwendet wird, in dem die Berechnung der Arkustangens- oder Arkuskotangensfunktion nicht effizient implementiert ist und deshalb lange dauert, können diese Funktionen in Schritt 65 mit einer geringen Genauigkeit berechnet werden. Eine hohe Genauigkeit ist erst erforderlich, wenn die Bedingung in Schritt 81 erfüllt ist, wenn also der ganzzahligen Anteil von $\tau_B = \tau_M$ bekannt ist. Beim Prototypen beträgt S=16, sodass in einen Quadranten lediglich 4 Schwellenwerte gelegt werden müssen, um den ganzzahligen Anteil von Index $\tau_B$ zu bestimmen. Dies zeigt, dass die Berechnung der Arkustangens- oder Arkuskotangensfunktion mit der in Schritt 65 erforderlichen Genauigkeit mittels einer Lookup-Tabelle implementiert werden kann.

Wie oben erwähnt, führt diese Iteration lediglich zu einem lokalen Maximum von $\varphi_{WS}$, das nicht notwendigerweise gleich dem globalen Maximum von $\varphi_{WS}$ entspricht. Auch ist der Abstand zweier lokaler Maxima nicht immer exakt gleich der Periodendauer des Ultraschalls, weil die Exponentialfunktion die Sinus- und Kosinusfunktion verzerrt. Insofern kann die Maximumsuche dahingehend modifiziert werden, dass abwechselnd ein Iterationsschritt gemäß Gleichung (34) und eine Überprüfung der beiden benachbarten lokalen Maxima durchgeführt wird. Die beiden Korrelationskoeffizienten $\varphi_{WC}$ und $\varphi_{WS}$ wurden an der Stelle $\tau_S$ berechnet. Bei round($\tau_B$) werden die Korrelationskoeffizienten erst im nächsten Iterationsschritt berechnet. Die Funktionen round() rundet eine Fließkommazahl auf die nächste ganze Zahl. Da $\tau_S$ anfangs lediglich eine Überlappung zwischen dem Ultraschallpaket und den Wavelets sicherstellen muss, kann $\varphi_{WS}$ am Anfang der Iterationen auch negativ sein. Insofern bietet sich an, zur Maximumsuche den Betrag $B(\tau)$ zu verwenden, dessen Berechnung beispielhaft in Schritt 67 dargestellt ist:

$$B(\tau) = \sqrt{\varphi_{WS}{}^2 + \varphi_{WC}{}^2} \tag{38}$$

Ein Betrag wird aber auch in Schritten 73, 75, 77 und 79 berechnet, auch wenn dies an anderen Stellen erfolgt.

Die Zahl S, die einen gebrochenen Anteil aufweisen kann, gibt die Zahl von Messwerten in einer Ultraschallperiode an. Deshalb sollen die Beträge $B(\text{round}(\tau_S \pm S))$ in Schritten 73 und 77 berechnet und mit dem Betrag $B(\tau_S)$ verglichen werden. Falls $B(\text{round}(\tau_S \pm S)) > B(\tau_S)$, kann als Schätzwert gemäß den Schritten 76 bzw. 80 in Verbindung mit Schritt 68 für die nächste Iteration $\tau_{S+} = \text{round}(\tau_B \pm S)$ verwendet werden.

In einer weiteren Ausführungsform können, wenn der Betrag $B(\text{round}(\tau_S \pm S))$ größer als der Betrag $B(\tau_S)$ ist, in dieser Richtung weitere Stellen round($\tau_S \pm nS$) hinsichtlich des Betrags $B(\text{round}(\tau_S \pm nS))$ der Korrelationsfaktoren in Schritten 75 bzw. 79 untersucht werden. Diese Stellen liegen um ein ganzzahliges Vielfaches der Periodendauer des Ultraschalls von $\tau_s$ entfernt. Ziel der Schritte 75 und 76 ist es, möglichst bereits bei der nächsten Iteration beim globalen Maximum zu landen.

Solche Feinheiten sind bei einer hohen Messrate der Flussgeschwindigkeit für die bei menschlicher Atmung typischerweise maximal zu erwartenden Beschleunigungen im Gasstrom weniger wichtig. Dann unterscheiden sich die Phasenlagen zweier Ultraschallpakete zweier aufeinander folgender Messungen wenig. Deshalb liegt $\tau_M$ bereits ausreichend nah bei $\tau_{M,vorig}$, der Stelle des Maximums bei der vorhergehenden Messung, so dass, wenn $\tau_{M,vorig}$ als erster Schätzwert verwendet wird, lediglich ein Iterationsschritt nötig ist, um $\tau_M$ zu bestimmen. In einem nicht ganz so optimalen Fall, wenn die Bedingung in Schritt 81 zunächst nicht erfüllt ist, sind zwei Iterationen notwendig, wobei die erste Iteration den ganzzahligen Anteil von $\tau_M$ bestimmt. Nachdem durch die erste Iteration die exponentiellen Hüllkurven des Ultraschallpakets und der beiden Motherwavelets optimal zur Deckung gebracht wurden, wird während der zweiten Iteration der gebrochene Anteil der Fließkommazahl $\tau_M$ exakt bestimmt. Ein Wechsel von einem lokalen zum globalen Maximum ist dann idealerweise nicht notwendig, was in Schritt 71 verifiziert werden kann.

Um festzustellen, ob sich ein Schätzwert $\tau_S$ oder ein verbesserter Schätzwert $\tau_B$ sich bereits in der richtigen Ultraschallperiode befindet, kann der Betrag $B(\tau_S)$ in Schritt 71 mit einem Grenzwert $C_B$ verglichen werden.

$$B(\tau_S) > C_B \tag{39}$$

Dieser Betrag muss über dem Grenzwert $C_B$ liegen, der von der Flussgeschwindigkeit und damit vom Laufzeitunterschied $\Delta t$ aber auch von anderen Parametern wie dem Alter der Ultraschallwandler abhängen kann. Aufgrund des Intensitätsabfalls bei hohen Flüssen wird $C_B$ mit zunehmendem Fluss abnehmen.

Eine weitere Möglichkeit, bei dem WaveTraX Algorithmus die richtige Periode zu bestimmen, besteht in einer Kombination mit dem TriTraX Algorithmus. So kann zunächst der Index $j_{93}$ bestimmt werden, dessen Ordinatenwert $W_{j93}$ am nächsten bei 0,93*M liegt. Hierbei ist M das Maximum der Messdaten, die gerade ausgewertet werden (vgl. Schritt 51). Als Schätzwert für den Index $j_M$ des Maximums kann round($\tau_B$) in Schritt 59 verwendet werden, wobei $\tau_B$ aus der vorangehenden Messung stammt. Nach Bestimmung von $j_{93}$ kann der erster Schätzwert $\tau_S$ aus

$$\tau_S = j_{93} + \frac{S}{8} \tag{40}$$

berechnet werden. Der WaveTraX Algorithmus läuft dann in Schritten 63 und 64 weiter.

**P-SEX Algorithmus**

Es hat sich auch der WaveTraX Algorithmus insbesondere bei einer wesentlichen Reduzierung der Signalamplitude und Signalverzerrungen aufgrund von Turbulenzen bei hohen Flüssen als nur unzureichend robust herausgestellt. Daher wurde ein neuer Algorithmus mit der Bezeichnung P-SEX (Phase-Shifted Excitation and Cross Correlation (X)) entwickelt.

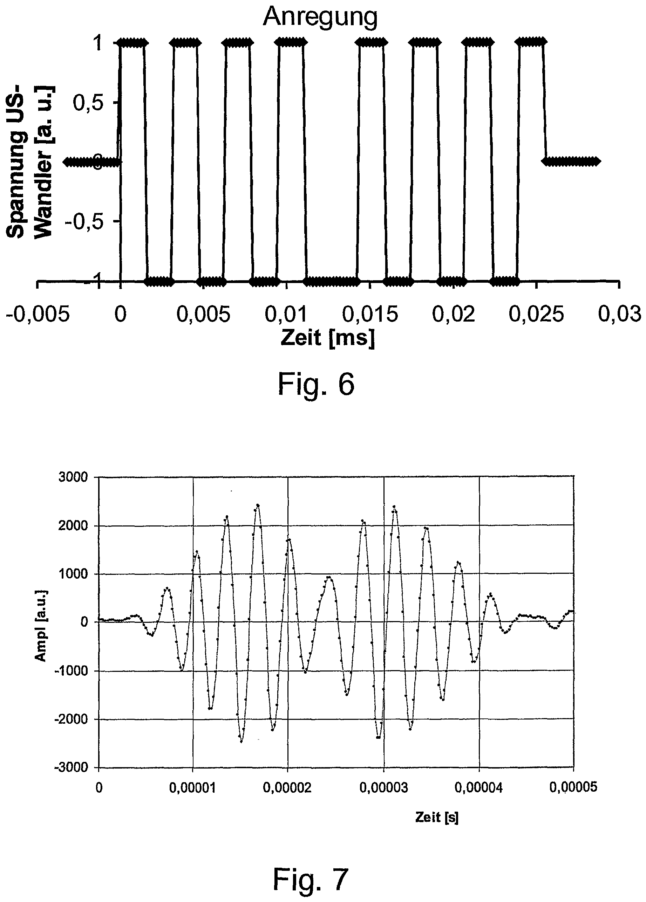

Die Anregung der Ultraschallwandler erfolgt über eine in Figur 6 dargestellte rechteckförmige Wechselspannung, deren Phase sich nach der Hälfte der Anregungszeit um 180° verschiebt oder invertiert wird, was äquivalent ist. Insgesamt umfasst die Wechselspannung acht Perioden. Die Frequenz der Wechselspannung $f_{us}$ beträgt wieder 312,5kHz und entspricht damit etwa der Resonanzfrequenz der Ultraschallwandler.

Das bei ruhendem Medium empfangene Signal, das beispielhaft in Figur 7 dargestellt ist, weißt ebenfalls einen Phasensprung auf. Man kann sich das empfangene Signal aus zwei zueinander phasenverschobenen Hälften vorstellen. Wird das Signal bei höheren Flüssen schlechter, so ist der Übergangsbereich der Phase nicht mehr wohl definiert.

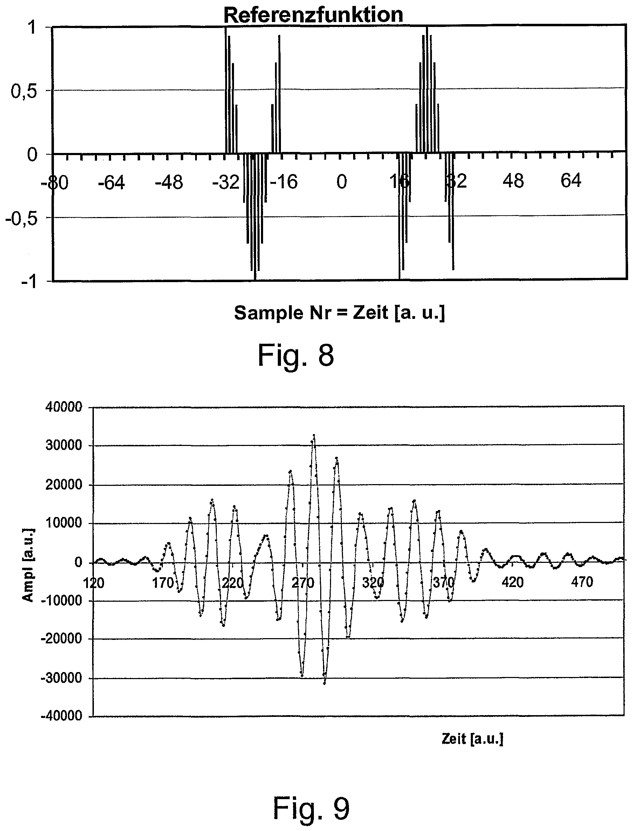

1. Daher wird als Referenzfunktion für die Berechnung einer Kreuzkorrelationsfunktion nicht wie beim WaveTraX Algorithmus eine Funktion verwendet, die dem empfangenen Signal ähnelt. Vielmehr wird die in Figur 8 dargestellte Referenzfunktion verwendet, bei der der Übergangsbereich "ausgeschnitten" ist. Es wurde durch Simulationen gefunden, dass eine in Figur 8 dargestellte Referenzfunktion mit einer Periode cos, 2 Perioden Null, einer Periode -cos eine in Figur 9 dargestellte Kreuzkorrelationsfunktion mit dem empfangenen Signal liefert, bei der das globale Maximum um etwa 23% über dem nächstniedrigerem Maximum liegt. Dadurch springt der P-SEX Algorithmus nicht mehr so leicht auf ein falsches Maximum. Bei der bei den Algorithmen TiDO, TriTraX und WaveTraX verwendeten Anregung von 5 Perioden, wodurch sich eine in Fig. 5 dargestellte, typische Signalform des empfangen Signals ergibt, beträgt der Unterschied gerade 1%.

2. Noch besser ist es, das linke der beiden betragsmäßig größten Minima, das im Folgenden als linkes Minimum $\tau_{IM}$ bezeichnet wird, zu betrachten. Der Unterschied zum betragsmäßig nächstkleineren Minimum beträgt fast 50% im Gegensatz zu den 23% bei Betrachtung des Maximums.

Es wurde aber beobachtet, dass das Minimum links vom linken Minimum betragsmäßig sehr groß wird - insbesondere größer als der Betrag des (bei geringen Flüssen globalen) Minimums rechts des linken Minimums $\tau_{IM}$. Gleichzeitig führt die bei hohen Flüssen veränderte Form der P-SEX Kreuzkorrelationsfunktion dazu, dass das linke Minimum $\tau_{IM}$ zum globalen Minimum betragsmäßig anwächst und wohl definiert ist (Fig. 9).

Es wird also in der Kreuzkorrelationsfunktion KKF gemäß Gleichung (41) das globale Minimum $y_1$ an der Stelle $\tau$ gesucht. Dann werden die beiden benachbarten Minima $y_0$ und $y_2$ gemäß Gleichungen (42) beziehungsweise (43) an den Stellen $\tau$-S beziehungsweise $\tau$+S betrachtet. Bei geringen Flüssen ist $y_0$ das linke Minimum. Bei hohen Flüssen ist $y_1$ das zum globale Minimum angewachsene linke Minimum und $y_2$ das bei geringen Flüssen globale Minimum. Die Auswahl des linken Minimums $\tau_{IM}$ erfolgt in Gleichung (44).

$$y_1 = Min(KKF) = KKF(\tau) \tag{41}$$

$$y_0 = KKF(\tau - S) \tag{42}$$

$$y_2 = KKF(\tau + S) \tag{43}$$

$$\tau_{IM} = \begin{cases} \tau - S & \text{wenn} \quad y_0 < y_2 \\ \tau & \text{sonst} \end{cases} \tag{44}$$

Im Ergebnis sucht dieser Algorithmus prinzipiell das linke Minimum. Der Laufzeitunterschied, ab dem das linke Minimum zum globalen Minimum angewachsen ist, beträgt etwa $5\mu s$. Im Vergleich hierzu beträgt der Laufzeitunterschied bei einem maximalen Fluss von 20 l/s bei der aktuellen Dimensionierung des Rohrquerschnitts etwa $24\mu s$. Damit lässt sich die grobe Position des Schallpakets gut bestimmen.

3. Zur exakten Phasenbestimmung wird in der Kreuzkorrelationsfunktion die cos-Periode ($-\pi \ldots \pi$), die auf das gesuchte Minimum folgt, ausgewählt:

$$W_i = KKF(\tau_M + i), \text{i=0, 1, 2, ... , S} \tag{45}$$

4. Die $W_i$ werden analog zu Schritten 54 und 55 des TriTraX Algorithmus nach einer Kosinus- bzw. Sinus-Funktion entwickelt, wobei die Koeffizienten $\varphi_C$ bzw. $\varphi_S$ berechnet werden.

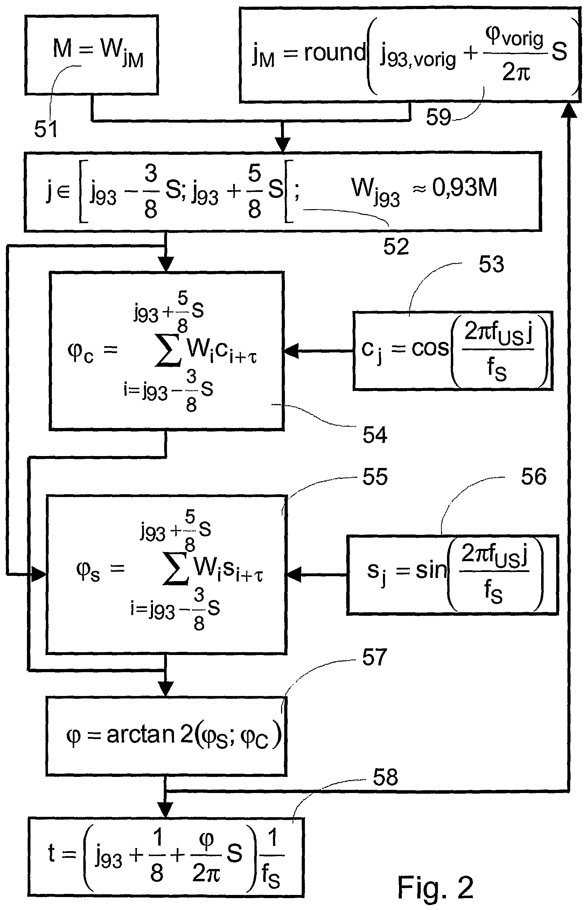

5. Anschließend wird entsprechend dem Schritt 57 in Figur 2 und Gleichung (25) mit einer arctan2-Funktion die Phase $\varphi$ berechnet. Dies ergibt eine Phasenbestimmung mit geringerem Fehler, als eine Periode aus dem Signal zu betrachten oder das Maximum der Kreuzkorrelationsfunktion durch eine Parabel anzufitten.

6. Schließlich wird die Laufzeit t aus Gleichung (46) berechnet:

$$t = \tau_M + \frac{\varphi}{2\pi f_{US}} \tag{46}$$

Neben den oben beschriebenen Algorithmen sind weitere Abwandlungen denkbar. Insbesondere kann das empfangene Ultraschallsignal mit einer Referenzfunktion korreliert oder gefaltet werden, die die Inverse des Übertragungswegs darstellt. Je nach Definition des Übertragungswegs, also beispielsweise Leistungsverstärker-Uftraschallwandler-Luft-Ultraschallwandler oder Triggerimpuls-Rechteckgenerator-Leistungsverstärker-Ultraschallwandler-Luft-Ultraschallwandler kann das Ergebnis der Faltung oder Korrelation beispielsweise ein Impuls oder eine Rechteck-Wechselspannung sein. Besonders vorteilhaft sind auch Korrelationsfunktionen, die das empfangene Ultraschallsignal näherungsweise in eine Dirac'sche Deltafunktion verwandeln, wobei idealerweise nur der Wert eines Zeitintervalls nennenswert von Null verschieden ist.

Zur Bestimmung des richtigen Intervalls kann eine andere Korrelationsfunktion eingesetzt werden als zur Bestimmung der Phase.

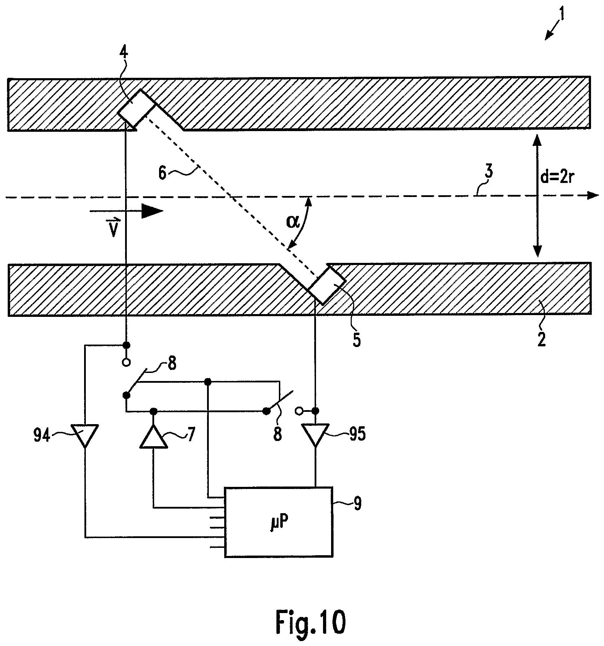

Fig. 10 zeigt eine Bauform eines bekannten Spirometers aus Fig. 11 mit einer erfindungsgemäßen Beschaltung. Herzstück der Steuerung ist der Mikroprozessor 9. Als Mikroprozessor kann insbesondere ein digitaler Signal Prozessor eingesetzt werden, der die Berechnung von Summenprodukten besonders schnell durchführen kann. Solche Summenprodukte kommen beispielsweise in Gleichungen (16), (29), (31), (35) und (36) vor. Der Mikroprozessor 9 steuert insbesondere Treiberschaltung 7, die die Wechselspannung für die beiden Ultraschallwandler 4 und 5 erzeugt. Da beide Wandler gleichzeitig senden sollen, ist lediglich eine Treiberschaltung erforderlich.

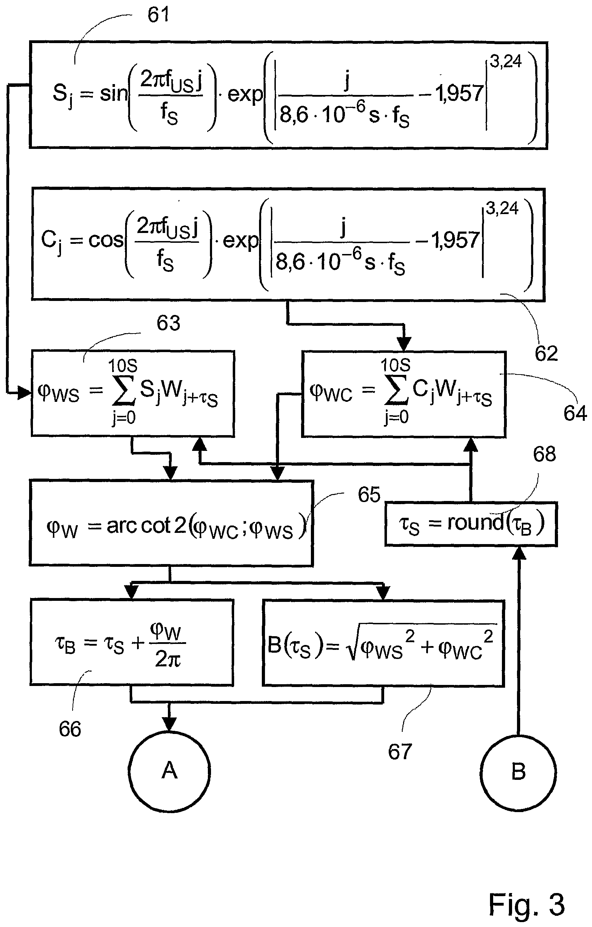

Insbesondere während des Empfangsbetriebs müssen die Wandler von der Treiberschaltung getrennt werden, was beispielsweise durch die Schalter 8 erfolgen kann, die aktiv durch den Mikroprozessor 9 ein-und ausgeschaltet werden. Diese Trennung kann auch dadurch erfolgen, dass der Ausgang der Treiberschaltung 7 hochohmig geschaltet wird. Eine dritte Möglichkeit besteht im Einsatz nichtlineare Bauelemente wie Dioden, wie er beispielsweise in EP 0 234 515 A1, Fig. 3 offenbart ist.

Im Empfangsbetrieb werden die von den Ultraschallwandlern gelieferten elektrischen Signale durch die Analog-Digital-Wandler 94 und 95 digitalisiert und dem Mikroprozessor 9 zur weiteren Verarbeitung zugeführt. Bei einem Prototypen wurden DSPs der Baureihe TMS320C24x (Texas Instruments) eingesetzt, die einen 12-Bit ADC und eine 32-Bit CPU enthalten.

Insbesondere das Rohr 2 wird nicht als wesentlicher Teil der Erfindung angesehen, weil es selbst oder zumindest ein Einsatzrohr wegen der besseren Desinfizierbarkeit auswechselbar ausgeführt ist und deshalb getrennt vom Spirometer vertrieben wird. Außerdem kann die mittlere Flussgeschwindigkeit in Richtung der durch die Wandler 4 und 5 festgelegten Achse 6 ein sinnvolles Messsignal für ein Anemometer sein, ohne dass eine Fläche spezifiziert ist und folglich kein Volumenstrom bestimmt werden kann.

Obwohl die Lage der ausgesandten Ultraschallpakete leicht zu bestimmen ist, weil ja der Mikroprozessor 9 die Treiberschaltung 7 triggert, soll betont werden, dass das technische Problem darin besteht, den Laufzeitunterschied $\Delta t$, weniger die Laufzeiten $t_{45}$ und $t_{54}$ oder die mittlere Laufzeit to, exakt zu bestimmen.

Aufgrund der in der Zwischenzeit zur Verfügung stehenden Ultraschallwandler können kurze Ultraschallimpulse erzeugt werden. So beträgt die Länge des in Fig. 5 dargestellten Ultraschallpakets 20 bis 30$\mu$s. Das Abklingverhalten nach einem Ultraschallimpuls ist so gut, dass der gleiche Ultraschallwandler nach Verstreichen der Laufzeit eines Ultraschallpakets von etwa 160$\mu$s nach dem Aussenden eines Ultraschallimpulses für den Empfang eines Ultraschallpakets verwendet werden kann. Deshalb werden bei einer Ausführungsform gleichzeitig von beiden Ultraschallwandlern 4 und 5 oder 14 und 15 Ultraschallimpulse ausgesendet und etwa 160$\mu$s später von beiden Ultraschallwandlern wieder empfangen. So liegen die Messpunkte $W_j$ für je eine Laufzeitmessung in beide Richtungen bereits nach gut 160$\mu$s vor, so dass man aus diesen Messdaten bereits den Laufzeitunterschied $\Delta t$ und die mittlere Laufzeit to berechnen kann.

Soweit ausreichend Speicher und Rechenleistung zur Verfügung stehen, können sofort nach der Aufzeichnung von zwei ersten Ultraschallpaketen die beiden nächsten Ultraschallpakete gesendet werden. Während der Laufzeit der nächsten Ultraschallpakete kann die Auswertung der Aufzeichnung der ersten Ultraschallpakete erfolgen. Die Flussgeschwindigkeit, die aus den beiden ersten Ultraschallpaketen berechnet wird, kann dann kurz vor der Aufzeichnung der beiden nächsten Ultraschallpakete zur Verfügung stehen. So können die oben angegebenen Abtastraten von 6,25 kHz für die Geschwindigkeitsmessung erreicht werden.

Bei jeder der oben beschriebenen Ausführungsformen kann als Plausibilitätskontrolle der Unterschied der aus zwei aufeinander folgenden Messungen bestimmten Geschwindigkeit ausgewertet werden. Dieser muss betragsmäßig kleiner als eine maximale Änderungsrate der Geschwindigkeit multipliziert mit dem zeitlichen Abstand der beiden Messungen liegen. Falls dies nicht der Fall ist, kann eine Fehlermeldung ausgegeben werden und/oder der jüngere Messwert verworfen werden.

Obwohl die bevorzugte Ausführungsform oben im Zusammenhang mit Luft beschrieben wurde, kann die Erfindung bei jedem anderen Fluid eingesetzt werden, um eine Flussgeschwindigkeit oder ein Volumenstrom zu bestimmen.

**Mathematische Symbole:**

| | |
|---|---|
| $\alpha$: | Winkel zwischen Achsen 3 und 6 |
| c: | Schallgeschwindigkeit im ruhenden Medium |
| $C_B$, $C_1$, $C_2$: | Konstanten |
| $S_j$, $C_j$: | "Abtastwerte" des Mothersinus- und -cosinuswavelets |
| $f_{US}$: | Ultraschallfrequenz |
| $f_S$: | Abtastrate |
| f(t), g(t): | zeitabhängige Funktionen |
| j: | Index der Messwerte |
| $j_M$: | Index von M |
| L: | Abstand der Ultraschallwandler |
| M: | Maximum der $W_j$ |

$\varphi_C$, $\varphi_S$:     Korrelationskoeffizienten

$\varphi_{WS}$, $\varphi_{WC}$:     Korrelationskoeffizienten

$\varphi_W$:     Phase

$\Delta t$:     Laufzeitunterschied

$t_0$:     mittlere Laufzeit

$t_{45}$, $t_{54}$     Schalllaufzeiten

$\Phi$:     Kreuzkorrelationsfunktion

r:     Radius von Rohr 2

S:     $f_S/f_{US}$ (in einer Ausführungsform 16)

v:     Strömungsgeschwindigkeit des Mediums

$\dot{V}$ :     Volumenstrom

$W_j$     Messwerte

$Y_T$:     zeitdiskrete zyklische Variante

$\tau_M$:     (interpolierter) Index

$\tau_S$:     (ganzzahliger) Schätzwert für $\tau_M$

$\tau_B$:     verbesserter Schätzwert für $\tau_M$

**Bezugszeichenliste**

| | |
|---|---|
| 1, 11 | Flussmessgerät |
| 2 | Rohr |
| 3 | Achse |
| 4, 5, 14, 15 | Ultraschallwandler |
| 6 | Achse |
| 7 | Treiberschaltung |
| 8 | Schalter |
| 9 | Mikroprozessor |
| 94, 95 | Analog-Digital-Wandler |
| 12 | waagrechten Schenkel |
| 13 | Achse |
| 17, 18 | senkrechter Schenkel |
| 31 | Schallpaket |
| M1, M2, M3, M4, M5, M6, M7, M8 | Maxima |
| 41-83 | Schritte |

**Patentansprüche**

**1.** Verfahren zur Bestimmung der zeitlichen Lage eines Wellenpakets (31) in einem Flussmessgerät, insbesondere Spirometer mit:

Abtasten (94, 95) des Wellenpakets zu einer Vielzahl von Zeitpunkten, wobei ein Messwert zu jedem Zeitpunkt entsteht;
Berechnen einer Summe von Produkten (43, 54, 55, 63, 64), wobei jedes Produkt für einen bestimmten Zeitpunkt aus der Vielzahl von Zeitpunkten berechnet wird und jedes Produkt das Produkt aus einem Wert einer Vergleichsfunktion (44, 53, 56, 61, 62) zu dem bestimmten Zeitpunkt und dem Messwert zu dem bestimmten Zeitpunkt ist; und
Berechnen (46, 47, 57, 58, 65 - 81) der zeitlichen Lage des Wellenpakets aus der Summe von Produkten
**gekennzeichnet durch**:

Ansteuern des Ultraschallwandlers, der das Wellenpaket erzeugt, mit einem Wechselspannungssignal, das einen Phasensprung aufweist.

**2.** Verfahren gemäß Anspruch 1, **dadurch gekennzeichnet, dass** die Vergleichsfunktion eine Sinusfunktion (56) ist, dass
eine zweite Summe von zweiten Produkten berechnet wird (54), wobei jedes zweite Produkt für einen bestimmten Zeitpunkt aus der Vielzahl von Zeitpunkten berechnet wird und jedes zweite Produkt das Produkt aus einem Wert einer Kosinusfunktion (53) zu dem bestimmten Zeitpunkt und dem Messwert zu dem bestimmten Zeitpunkt ist, und dass

die zeitliche Lage des Wellenpakets als Arcustangensfunktion (57) des Quotienten aus der Summe von Produkten dividiert durch die zweite Summe von zweiten Produkten berechnet wird.

3. Verfahren gemäß einem der obigen Ansprüche, **gekennzeichnet durch**:

Bestimmen des Messpunkts, an dem das Wellenpaket zum ersten Mal einen vorgegebenen Bruchteil des Maximums aller Messpunkte des Wellenpakets überschreitet.
Auswählen (52) einer Periode des Wellenpakets um den bestimmten Messpunkt herum zur Berechnung der Summen von Produkten.

4. Verfahren gemäß Anspruch 1 oder 2 , **gekennzeichnet durch**:

Bestimmen des Messpunkts, an dem das Wellenpaket zum ersten Mal einen vorgegebenen Bruchteil des Minimums aller Messpunkte des Wellenpakets unterschreitet.
Auswählen einer Periode des Wellenpakets um den bestimmten Messpunkt herum zur Berechnung der Summen von Produkten.

5. Verfahren gemäß einem der Ansprüche 1, 2, 3 oder 4, **gekennzeichnet durch**:

Berechnen (43) einer Vielzahl von Summen von Produkten, wobei jeweils unterschiedliche Vergleichsfunktionen, aber die gleichen Messwerte verwendet werden, wobei die Vergleichsfunktionen um eine jeweils unterschiedliche Zeitdifferenz verschoben, sonst aber gleich sind;
Bestimmen (45) des Maximums der Summen von Produkten; und
Berechnen der zeitlichen Lage des Wellenpakets aus der Zeitdifferenz, um die die Vergleichsfunktion verschoben wurde, die zur Berechnung der maximalen Summe von Produkten verwendet wurde.

6. Verfahren gemäß Anspruch 5, **gekennzeichnet durch**:

Legen (46) einer Parabel durch die maximale Summe von Produkten sowie die beiden benachbarten Summen, die aus Vergleichsfunktionen berechnet werden, die um den zeitlichen Abstand zweier benachbarter Messpunkte gegenüber der Vergleichsfunktion der maximalen Summe nach vorne und nach hinten verschoben sind; und
Berechnen (47) der zeitlichen Lage des Wellenpakets aus der Lage des Scheitelpunkts der Parabel.

7. Verfahren gemäß einem der obigen Ansprüche, **dadurch gekennzeichnet, dass** die Vergleichsfunktion aus einer Kosinus-Periode, zwei Perioden null, einer inversen Kosinus-Periode und sonst null besteht.

8. Flussmessgerät(1, 11) mit:

einem ersten Schallwandler (4, 14);
einem zweiten Schallwandler (5, 15), wobei die beiden Schallwandler aufeinander ausgerichtet sind, so dass der zweite Schallwandler (5, 15) einen Teil des vom ersten Schallwandler (4, 14) erzeugten Schalls aufnimmt und umgekehrt, wobei die beiden Schallwandler ein elektrisches Signal in Schall und umgekehrt wandeln;
einer Treiberschaltung (7), die mit dem ersten Schallwandler (4, 5, 14, 15) verbunden ist, und den ersten Schallwandler zeitweise mit Wechselspannung zur Erzeugung von Schall versorgt;
einen Analog-Digital-Wandler (94, 95) zur Abtastung und Digitalisierung des vom zweiten Schallwandler ausgegebenen elektrischen Signals zu einer Vielzahl von Zeitpunkten, wobei ein Messwert zu jedem Zeitpunkt entsteht, wobei der Eingang des Analog-Digital-Wandlers (94, 95) mit dem zweiten Schallwandler (4, 5, 14, 15) verbunden ist,
einen Mikroprozessor (9), der mit dem Ausgang des Analog-Digital-Wandlers (94, 95) verbunden ist und dem die Messwerte zugeführt werden, wobei der Mikroprozessor (9) die folgenden Schritte ausführt:

Berechnen einer Summe von Produkten (43, 54, 55, 63, 64), wobei jedes Produkt für einen bestimmten Zeitpunkt aus der Vielzahl von Zeitpunkten berechnet wird und jedes Produkt das Produkt aus einem Wert einer Vergleichsfunktion (44, 53, 56, 61, 62) zu dem bestimmten Zeitpunkt und dem Messwert zu dem bestimmten Zeitpunkt ist; und

Berechnen (46, 47, 57, 58, 65 - 81) der zeitlichen Lage des Wellenpakets aus der Summe von Produkten

**dadurch gekennzeichnet, dass** die Wechselspannung, mit der die Treiberschaltung (7) den ersten Schallwandler zeitweise mit Wechselspannung versorgt, einen Phasensprung aufweist.

9. Flussmessgerät gemäß Anspruch 8, **dadurch gekennzeichnet, dass** die Vergleichsfunktion eine Sinusfunktion (56, 61) ist, dass der Mikroprozessor (9)

eine zweite Summe von zweiten Produkten berechnet (54, 64), wobei jedes zweite Produkt für einen bestimmten Zeitpunkt aus der Vielzahl von Zeitpunkten berechnet wird und jedes zweite Produkt das Produkt aus einem Wert einer Kosinusfunktion (53, 62) zu dem bestimmten Zeitpunkt und dem Messwert zu dem bestimmten Zeitpunkt ist, und

die zeitliche Lage des Wellenpakets als Arcustangensfunktion (57, 56) des Quotienten aus der Summe von Produkten dividiert durch die zweite Summe von zweiten Produkten berechnet.

## Claims

1. Method for determining the temporal position of a wave packet (31) in a flow measuring apparatus, in particular a spirometer, comprising:

sampling (94, 95) the wave packet at a plurality of points in time, wherein a measured value is created at each point in time;

calculating a sum of products (43, 54, 55, 63, 64), wherein each product is calculated for a determined point in time from a plurality of points in time and each product is the product of a value of a compare function (44, 53, 56, 61, 62) at said determined point in time and the measured value at said determined point in time; and

calculating (46, 47, 57, 58, 65 - 81) the temporal position of the wave packet from the sum of products **characterized by**:

driving the ultrasonic transducer generating the wave packet by an AC signal having a phase jump.

2. Method according to claim 1, **characterized in that** the compare function is a sine function (56), that

a second sum of second products is calculated (54), wherein each second product is calculated for a determined point in time from a plurality of points in time and each second product is the product of a value of a cosine function (53) at said determined point in time and the measured value at said determined point in time, and that

the temporal position of the wave packet is calculated as arc tangent function (57) of the quotient from the sum of products divided by the second sum of second products.

3. Method according to one of claims above, **characterized by**:

determining the measuring point at which the wave packet exceeds for the first time a predetermined fraction of the maximum of all measuring points of the wave packet;

choosing (52) a period of the wave packet around the determined measuring point so as to calculate the sums of products.

4. Method according to claim 1 or 2, **characterized by**:

determining the measuring point at which the wave packet drops for the first time below a predetermined fraction of the minimum of all measuring points of the wave packet;

choosing a period of the wave packet around the determined measuring point so as to calculate the sums of products.

5. Method according to one of claims 1, 2, 3 or 4, **characterized by**:

calculating (43) a plurality of sums of products, wherein different compare functions, but the same measured values are used, wherein the compare functions are each shifted by a different time difference, but are equal otherwise;

determining (45) the maximum of the sums of products; and
calculating the temporal position of the wave packet from the time difference by which the compare function was shifted, which was used to calculate the maximum sum of products.

6. Method according to claim 5, **characterized by**:

laying (46) a parabola through the maximum sum of products and the two adjacent sums which are calculated from compare functions, which are shifted forward and backward by the time interval of two adjacent measuring points with respect to the compare function of the maximum sum; and
calculating (47) the temporal position of the wave packet from the position of the vertex of the parabola.

7. Method according to one of the claims above, **characterized in that** the compare function consists of a cosine period, two periods zero, an inverse cosine period and otherwise zero.

8. Flow measuring apparatus (1, 11), comprising:

a first sonic transducer (4, 14);
a second sonic transducer (5, 15), wherein the two sonic transducers are aligned with respect to each other so that the second sonic transducer (5, 15) absorbs a part of the sound generated by the first sonic transducer (4, 14) and vice versa, wherein the two sonic transducers convert an electric signal to sound and vice versa;
a driver circuit (7) which is connected to the first sonic transducer (4, 5, 14, 15) and temporarily supplies the first sonic transducer with an alternating voltage to generate sound;
an analog-digital converter (94, 95) for sampling and digitalizing the electric signal emitted by the second sonic transducer at a plurality of points in time, wherein a measured value is created at each point in time, wherein the input of the analog-digital converter (94, 95) is connected to the second sonic transducer (4, 5, 14, 15),
a microprocessor (9) connected to the output of the analog-digital converter (94, 95) and to which the measured values are supplied, said microprocessor (9) performing the following steps:

calculating a sum of products (43, 54, 55, 63, 64), wherein each product is calculated for a determined point in time from the plurality of points in time and each product is the product of a value of a compare function (44, 53, 56, 61, 62) at said determined point in time and the measured value at said determined point in time; and
calculating (46, 47, 57, 58, 65 - 81) the temporal position of the wave packet from the sum of products

**characterized in that** the alternating voltage which the driver circuit (7) temporarely supplies to the first sonic transducer has a phase jump.

9. Flow measuring apparatus according to claim 8, **characterized in that** the compare function is a sine function (56, 61), that the microprocessor (9)

calculates (54, 64) a second sum of second products, wherein each second product is calculated for a determined point in time from a plurality of points in time and each second product is the product of a value of a cosine function (53, 62) at said determined point in time and the measured value at said determined point in time, and calculates the temporal position of the wave packet as arc tangent function (57, 56) of the quotient from the sum of products divided by the second sum of second products.

**Revendications**

1. Procédé pour déterminer la position temporelle d'un paquet d'ondes (31) dans un appareil de mesure de flux, notamment un spiromètre, avec :

l'analyse (94, 95) du paquet d'ondes à une pluralité de moments, une valeur de mesure étant produite à chaque moment ;
le calcul d'une somme de produits (43, 54, 55, 63, 64), chaque produit étant calculé pour un moment défini à partir de la pluralité de moments et chaque produit étant le produit d'une valeur d'une fonction de comparaison (44, 53, 56, 61, 62) par rapport au moment défini et à la valeur de mesure par rapport au moment défini ; et
le calcul (46, 47, 57, 58, 65 - 81) de la position temporelle du paquet d'ondes à partir de la somme de produits ;

**caractérisé par** :

l'excitation du transducteur ultrasonique produisant le paquet d'ondes, avec un signal de tension alternative, comportant un saut de phase.

**2.** Procédé selon la revendication 1, **caractérisé en ce que** :

la fonction de comparaison est une fonction sinus (56) ;
une deuxième somme de deuxièmes produits est calculée (54), chaque deuxième produit étant calculé pour un moment défini à partir de la pluralité de moments et chaque deuxième produit étant le produit d'une valeur d'une fonction cosinus (53) par rapport au moment défini et à la valeur de mesure par rapport au moment défini ; et la position temporelle du paquet d'ondes est calculée à partir de la fonction d'arc tangente (57) du quotient à partir de la somme de produits divisée par la deuxième somme de deuxièmes produits.

**3.** Procédé selon l'une quelconque des revendications précédentes, **caractérisé par** :

la détermination du point de mesure auquel le paquet d'ondes dépasse pour la première fois une fraction prédéfinie du maximum de tous les points de mesure du paquet d'ondes ;
la sélection (52) d'une période du paquet d'ondes autour du point de mesure défini pour calculer les sommes de produits.

**4.** Procédé selon la revendication 1 ou 2, **caractérisé par** :

la détermination du point de mesure auquel le paquet d'ondes passe pour la première fois en dessous d'une fraction prédéfinie du minimum de tous les points de mesure du paquet d'ondes ;
la sélection d'une période du paquet d'ondes autour du point de mesure défini pour calculer les sommes des produits.

**5.** Procédé selon l'une quelconque des revendications 1, 2, 3 ou 4, **caractérisé par** :

le calcul (43) d'une pluralité de sommes de produits, respectivement des fonctions de comparaison différentes mais utilisant les mêmes valeurs de mesure, les fonctions de comparaison étant décalées d'une différence de temps respectivement différente mais étant par ailleurs identiques ;
la détermination (45) du maximum des sommes de produits ; et
le calcul de la position temporelle du paquet d'ondes à partir de la différence de temps dont la fonction de comparaison est décalée et utilisée pour calculer la somme de produits maximale.

**6.** Procédé selon la revendication 5, **caractérisé par** :

la construction (46) d'une parabole à travers la somme maximale de produits ainsi que des deux sommes connexes calculées à partir des fonctions de comparaison décalées en avant et en arrière de la distance temporelle de deux points de mesure connexes par rapport à la fonction de comparaison de la somme maximale ; et
le calcul (47) de la position temporelle du paquet d'ondes à partir de la position du sommet de la parabole.

**7.** Procédé selon l'une quelconque des revendications précédentes, **caractérisé en ce que** la fonction de comparaison se compose d'une période cosinus, de deux périodes à zéro, d'une période cosinus inversée et sinon à zéro.

**8.** Appareil de mesure de flux (1, 11) avec :

un premier transducteur acoustique (4, 14) ;
un deuxième transducteur acoustique (5, 15), les deux transducteurs acoustiques étant alignés l'un sur l'autre, de sorte que le deuxième transducteur acoustique (5, 15) reçoive une partie du son produit par le premier transducteur acoustique (4, 14) et inversement, les deux transducteurs acoustiques transformant un signal électrique en son et inversement ;
un circuit de commande (7) relié au premier transducteur acoustique (4, 5, 14, 15) et alimentant de temps en temps le premier transducteur acoustique en tension alternative pour produire un son ;
un convertisseur analogique-numérique (94, 95) servant à l'analyse et à la numérisation du signal électrique envoyé par le deuxième transducteur acoustique à une pluralité de moments, une valeur de mesure étant produite à chaque moment, l'entrée du convertisseur analogique-numérique (94, 95) étant reliée au deuxième transducteur acoustique (4, 5, 14, 15) ;

un microprocesseur (9) relié à la sortie du convertisseur analogique-numérique (94, 95) et auquel les valeurs de mesure sont amenées, le microprocesseur (9) réalisant les étapes suivantes :

calcul d'une somme de produits (43, 54, 55, 63, 64), chaque produit étant calculé pour un moment défini à partir de la pluralité de moments et chaque produit étant le produit d'une valeur d'une fonction de comparaison (44, 53, 56, 61,62) par rapport au moment défini et à la valeur de mesure par rapport au moment défini ; et

le calcul (46, 47, 57, 58, 65 - 81) de la position temporelle du paquet d'ondes à partir de la somme de produits ; **caractérisé en ce que** la tension alternative avec laquelle le circuit de commande (7) alimente de temps en temps le premier transducteur acoustique comporte un saut de phase.

9. Appareil de mesure de flux selon la revendication 8, **caractérisé en ce que** la fonction de comparaison est une fonction sinus (56, 61), que le microprocesseur (9) :

calcule une deuxième somme de deuxièmes produits (54, 64), chaque deuxième produit étant calculé pour un moment défini à partir de la pluralité de moments et chaque deuxième produit étant le produit d'une valeur d'une fonction cosinus (53, 62) par rapport au moment défini et à la valeur de mesure par rapport au moment défini ; et

la position temporelle du paquet d'ondes étant calculée comme la fonction d'arc tangente (57, 56) du quotient à partir de la somme de produits divisée par la deuxième somme de deuxièmes produits.

$$M = W_{j_M}$$

$$j_M = \text{round}\left(j_{93,\text{vorig}} + \frac{\varphi_{\text{vorig}}}{2\pi} S\right)$$

41

48

42

$$j \in \left[j_M - S/2 \,;\, j_M + S/2\right[$$

$$Y_\tau = \sum_{i=j_M - S/2}^{j_M + S/2 - 1} W_i f_{i+\tau}$$

$$f_i = \cos\left(\frac{2\pi f_{US} i}{f_S}\right)$$

43

44

45

$$\text{Max} = Y_{\tau_M}$$

$$P_\tau = a Y_\tau^2 + b Y_\tau + c; \quad \tau \in \left[\tau_M - 1, \tau_M, \tau_M + 1\right]$$

46

$$\tau_{\text{Max}} = -\frac{b}{2a}$$

47

## Fig. 1

$$M = W_{j_M}$$

51

$$j_M = \text{round}\left(j_{93,vorig} + \frac{\varphi_{vorig}}{2\pi}S\right)$$

59

$$j \in \left[j_{93} - \frac{3}{8}S; \; j_{93} + \frac{5}{8}S\right[; \qquad W_{j_{93}} \approx 0{,}93M$$

52

53

$$\varphi_c = \sum_{i=j_{93}-\frac{3}{8}S}^{j_{93}+\frac{5}{8}S} W_i c_{i+\tau}$$

$$c_j = \cos\left(\frac{2\pi f_{US}j}{f_S}\right)$$

54

55

56

$$\varphi_s = \sum_{i=j_{93}-\frac{3}{8}S}^{j_{93}+\frac{5}{8}S} W_i s_{i+\tau}$$

$$s_j = \sin\left(\frac{2\pi f_{US}j}{f_S}\right)$$

57

$$\varphi = \arctan 2(\varphi_S; \varphi_C)$$

58

$$t = \left(j_{93} + \frac{1}{8} + \frac{\varphi}{2\pi}S\right)\frac{1}{f_S}$$

Fig. 2

$$S_j = \sin\left(\frac{2\pi f_{US}j}{f_S}\right) \cdot \exp\left(\left|\frac{j}{8{,}6 \cdot 10^{-6}\,s \cdot f_S} - 1{,}957\right|^{3{,}24}\right)$$

61

$$C_j = \cos\left(\frac{2\pi f_{US}j}{f_S}\right) \cdot \exp\left(\left|\frac{j}{8{,}6 \cdot 10^{-6}\,s \cdot f_S} - 1{,}957\right|^{3{,}24}\right)$$

62

63

$$\varphi_{WS} = \sum_{j=0}^{10S} S_j W_{j+\tau_S}$$

$$\varphi_{WC} = \sum_{j=0}^{10S} C_j W_{j+\tau_S}$$

64

68

65

$$\varphi_W = \operatorname{arc\,cot}2\left(\varphi_{WC};\varphi_{WS}\right)$$

$$\tau_S = \operatorname{round}(\tau_B)$$

$$\tau_B = \tau_S + \frac{\varphi_W}{2\pi}$$

$$B(\tau_S) = \sqrt{\varphi_{WS}^2 + \varphi_{WC}^2}$$

66

67

( A )

( B )

Fig. 3

Fig. 4

Fig.5

## Anregung

**Fig. 6**

**Fig. 7**

**Referenzfunktion**

Sample Nr = Zeit [a. u.]

## Fig. 8

Zeit [a.u.]

## Fig. 9

Fig.10

**Fig.11**
(Stand der Technik)

**Fig.12**
(Stand der Technik)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 2275108 A
- EP 0051293 A1
- EP 0243515 A1
- EP 0597060 B1
- CH 669463 A5
- WO 0026618 A1
- EP 0713080 A1

- WO 9005283 A1
- EP 1279368 A2
- WO 9005283 A
- US 20020143479 A1
- EP 0797105 A2
- US 6305233 B1
- EP 0234515 A1

**In der Beschreibung aufgeführte Nicht-Patentliteratur**

- **ANTJE OHLHOFF.** Anwendung der Wavelettransformation in der Signalverarbeitung. Universität Bremen, 1996